(19) Europäisches Patentamt
European Patent Office
Office européen des brevets

(11) EP 4 506 387 A1

(12) **EUROPEAN PATENT APPLICATION**
published in accordance with Art. 153(4) EPC

(43) Date of publication:
**12.02.2025 Bulletin 2025/07**

(21) Application number: **23780571.8**

(22) Date of filing: **28.03.2023**

(51) International Patent Classification (IPC):
***C08F 290/06*** (2006.01) ***B33Y 70/00*** (2020.01)
***C08F 220/58*** (2006.01) ***C09D 4/00*** (2006.01)
***C09D 4/02*** (2006.01) ***C09D 11/00*** (2014.01)
***C09J 4/00*** (2006.01) ***C09J 4/02*** (2006.01)
***C09J 11/06*** (2006.01)

(52) Cooperative Patent Classification (CPC):
**B33Y 70/00; C08F 220/58; C08F 290/06; C09D 4/00; C09D 11/00; C09J 4/00; C09J 11/06**

(86) International application number:
**PCT/JP2023/012610**

(87) International publication number:
**WO 2023/190562 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.03.2022 JP 2022056587**

(71) Applicant: **KJ Chemicals Corporation**
**Tokyo 103-0023 (JP)**

(72) Inventors:
- **FURUSHO Haruka**
  **Yatsushiro-shi Kumamoto 866-0081 (JP)**
- **OKADA Kazuho**
  **Yatsushiro-shi Kumamoto 866-0081 (JP)**
- **YASUNAGA Atsushi**
  **Yatsushiro-shi Kumamoto 866-0081 (JP)**

(74) Representative: **ABG Intellectual Property Law, S.L.**
**Avenida de Burgos, 16D**
**Edificio Euromor**
**28036 Madrid (ES)**

# (54) CURABLE COMPOSITION CONTAINING WATER-INSOLUBLE MULTIFUNCTIONAL (METH) ACRYLAMIDE

(57) [Problem] To provide: a curable composition which has high curability and high transparency, while exhibiting good adhesion to various base materials, and which is used in both an aqueous system and an organic system and is applied to UV curing and/or thermal curing; a coating agent composition, a bonding agent composition, an adhesive composition, an ink composition, an aqueous ink composition, an ink composition for three-dimensional modeling, an aqueous coating material composition, a sealing agent composition, a nail cosmetic composition, a dental material and a decorative coating agent, each of which contains this curable composition; and cured products and molded articles of these materials. [Solution] A curable composition which contains: (A) a water-insoluble multifunctional (meth)acrylamide; and (B) a polymerizable compound other than the component (A).



Processed by Luminess, 75001 PARIS (FR)

## Description

### TECHNICAL FIELD

The present disclosure relates to a curable composition. containing one or more water-insoluble polyfunctional (meth)acrylamides.

### BACKGROUND ART

N-substituted or N, N-disubstituted (meth)acrylamides having two or more (meth)acrylamides in a molecule are called polyfunctional (meth)acrylamide, and are widely used as components of ultraviolet (UV)curable resins and thermosetting resins. Multifunctional (meth)acrylamides have a wide variety of structures depending on the type and number of their substituents, and can cover a wide range of properties and functions such as liquid to solid, hydrophilic (high-polarity) to hydrophobic (low-polarity), and low viscosity to high viscosity. Multifunctional (meth)acrylamides are used in a wide variety of fields such as inks, pressure-sensitive adhesives, adhesives, various coating agents and paints, nail cosmetics and dental materials, cosmetics and medical materials such as contact lenses, reactive diluents for ultraviolet (UV) curable resins, and crosslinking agents for thermal polymerization or photopolymerization.

In recent years, volatile organic chemical substances (VOC) have been regarded as a problem as one of the causes of air pollution, and it has been required to suppress the amount of VOC emissions in various fields. Therefore, aqueous (water-based) curable compositions which do not contain organic solvents or the like and in which water is used as a solvent, and active energy ray-curable compositions which can be cured with UV, EB (electron beam), etc., and do not contain an organic solvents or water have been attracting attention. In particular, aqueous curable compositions using water as a solvent require an extremely large amount of energy to dry the water, whereas active energy ray-curable compositions are greatly expected because of energy-saving and have a low environmental impact. Furthermore, water-soluble or water-dispersible water-based UV-curable resins have been actively studied because water can be evaporated by the heat generated as polymerization reaction (curing) proceeds by irradiation with active energy rays, and cured films can be rapidly obtained even with a small amount of energy by the simultaneous progress of UV curing and drying, and the viscosity of the composition can be adjusted and shrinkage during curing (curing shrinkage) can be suppressed by adjusting the water content.

### SUMMARY OF THE INVENTION

### PROBLEMS TO BE SOLVED BY THE INVENTION

The present disclosure provides a curable composition which has good wettability and adhesion to various substrates while having high curability and transparency, is liquid at room temperature, has excellent handleability, and can be used in both aqueous and organic systems, and is applicable to ultraviolet curing and/or thermal curing. The present invention also provides a coating agent composition, a pressure-sensitive adhesive composition, an adhesive composition, an ink composition, an aqueous ink composition, an ink composition for three-dimensional modeling, an aqueous coating composition, a sealant composition, a nail cosmetic composition, a dental material composition, a decorative coating agent composition, each of which contains the curable composition, as well as cured products and molded articles of these various compositions.

### SOLUTIONS TO THE PROBLEMS

It has been found that the above-mentioned problems can be solved by using a curable composition containing a water-insoluble polyfunctional (meth)acrylamide (A) and a polymerizable compound (B) other than (A).

### EFFECTS OF THE INVENTION

The curable composition of the present disclosure contains a water-insoluble polyfunctional (meth)acrylamide (A) and a polymerizable compound (B) and has high curability. Due to the interaction between (A) and (B), the curable composition potentially has high wettability and adhesion to various substrates from low-polarity resin-based materials to high-polarity glass and metallic materials, good compatibility with water, organic solvents from low-polarity to high-polarity, various general-purpose monomers, and the like, and has high transparency. The curable composition is a liquid at room temperature and has good bundling properties, and the viscosity thereof can be adjusted without limitation from a low viscosity to a high viscosity depending on the application. The curable composition of the present disclosure can be used in both aqueous and organic systems and in UV curing and/or thermal curing, and can be suitably used in various

applications such as printing ink compositions for various systems such as inkjet printing, screen-printing, offset printing, flexographic printing, and gravure printing, three-dimensional modeling ink compositions, coating agent compositions, pressure-sensitive adhesive compositions, adhesive compositions, paint compositions, sealant compositions, nail cosmetics, decorative coating agents used in decorative films and decorative sheets, and dental materials.

DESCRIPTION OF THE EMBODIMENTS

Hereinafter, the present invention will be described in detail. The scope of the present invention is not limited to the embodiments described herein, and various modifications can be made without departing from the spirit of the present invention. In addition, when multiple upper and lower limit values are listed for a particular parameter, any of these upper and lower limit values can be combined to set an appropriate numerical range.

One embodiment of the present disclosure is a curable composition (D) containing a polyfunctional (meth) acrylamide (A) and a polymerizable compound (B). The polyfunctional (meth)acrylamide (A) is a compound having two or more (meth)acrylamide groups in a molecule. The number of (meth)acrylamide groups in (A) is preferably 6 or less. When the number of (meth)acrylamide groups is more than 6, the acrylic equivalent of the compound (A) becomes small, so that the curable composition shrinks significantly during curing, and the obtained cured film becomes more likely to deformation or crack. In addition, when the number of (meth)acrylamide groups is more than 6, the viscosity and polarity of (A) increase due to the formation of hydrogen bonds between the (meth)acrylamide groups, which may deteriorate the handling of (A) and the compatibility between (A) and (B). From the same viewpoint, the number of (meth)acrylamide groups of (A) are more preferably 4 or less.

The polyfunctional (meth)acrylamide (A) is a compound which is water-insoluble by itself but has an amphiphilic property. (A) becomes water-soluble by coexisting with a water-soluble polymerizable compound (B), and becomes soluble in low-polarity organic solvent by coexisting with a water-insoluble polymerizable compound (B). Therefore, the curable composition (D) containing (A) and (B) is used for in both aqueous systems and organic systems. By adjusting the type and content of (A) and (B), the composition has high compatibility with water, organic solvents having polarity from low to high and various general-purpose monomers, etc., and the composition has high transparency. Further, when used as a water-based curable composition such as an aqueous ink or an aqueous coating material, the composition has good drying and curing properties, and exhibits the effects of maintaining transparency, smoothness, and glossiness without causing unevenness or cloudiness in the ink film (or image) or coating material film that has been dried by evaporating water. Furthermore, since the polyfunctional (meth)acrylamide (A) has one or more (meth)acrylamide groups in the molecule, and the polymerizable compound (B) has one or more polymerizable groups in the molecule, the curable composition (D) containing (A) and (B) has high curability to active energy rays such as ultraviolet rays (UV) and electronic rays (EB) and/or heat, and the obtained cured products are excellent in strength, hardness, heat resistance and the like.

One embodiment of the present disclosure is a curable composition (D) containing one or more polyfunctional (meth)acrylamides (A) and one or more polymerizable compounds (B) (excluding (A)), wherein the solubility parameter (SP value) of the polyfunctional (meth)acrylamide (A) is 8.8 to 11.0 $(call/cm^3)^{1/2}$. The SP value in the present disclosure is calculated by the Fedors method described in Polymer Engineer Science, Vol.14, P.147, Y.1974, and the unit is $(call/cm^3)^{1/2}$. A higher SP value of a compound indicates a higher hydrophilicity of the compound, whereas a lower SP value of a compound indicates a higher hydrophobicity of the compound. The SP value of the polyfunctional (meth) acrylamide (A) is 8.8 to 11.0 (hereinafter, units are omitted), and such (A) is amphiphilic that exhibits both of hydrophilicity and hydrophobicity. The curable composition (D) has high wettability, adhesion, adhesive strength, etc., for various substrates, from low-polarity resin materials to high-polarity glass and metal materials due to the amphiphilicity of (meth) acrylamide (A), the high cohesiveness of the (meth)acrylamide group, and the high adhesion to substrates derived from the (meth)acrylamide group. Coating compositions, pressure-sensitive adhesive compositions, ink compositions, and the like containing the curable composition (D) have high wettability, adhesiveness, and pressure-sensitive adhesiveness. In addition, adhesive compositions containing the curable composition (D) has high adhesive strength.

One embodiment of the present disclosure is the curable composition (D), wherein the SP value of the polymerizable compound (B) (excluding the polyfunctional (meth)acrylamide (A)) is from 8.5 to 14.5. The SP value of the polymerizable compound (B) is within this range, and (B) has hydrophilicity, hydrophobicity, or amphiphilicity depending on the type. Therefore, the compatibility between the polymerizable compound (B) and the polyfunctional (meth)acrylamide (A) is good, the transparency of the curable composition (D) containing (A) and (B) is high, and the transparency of the cured product obtained by curing (D) is also high. In addition, the curable composition (D) has good solubility in water and various organic solvents and is suitably used in both aqueous and organic systems. In the present disclosure, "water-based" and "aqueous" in the terms of water-based curable compositions, aqueous ink compositions, or aqueous coating compositions indicate that water accounts for 60% by mass or more of the total mass of volatile components in various compositions. Similarly, "organic-based" and "organic" in the terms of organic-based curable compositions, organic ink compositions, or organic coating compositions indicate that organic accounts for 60% by mass or more of the total of volatile components in various compositions. Hereinafter, when not described as aqueous or water-based, it refers to organic or organic-based. The various compositions containing the curable composition (D) described above can be obtained in either organic or aqueous system. In particular, in an aqueous ink composition and an aqueous coating composition, the curable composition (D) has good pigment dispersibility, so that the ink composition has excellent printing properties such as ejection stability and print clarity, and the coating film obtained by curing the coating composition has excellent surface properties such as surface smoothness and surface gloss.

One embodiment of the present disclosure is the various curable compositions (D) in which the absolute value of the difference in the SP value between the polyfunctional (meth)acrylamide (A) and the polymerizable compound (B) is 3.0 or less. The closer the SP values of A and B are, the closer their hydrophilicity and hydrophobicity are, and therefore the higher the compatibility between A and B is, and the more excellent the transparency of the curable composition (D) and the cured product of D is. From this viewpoint, the absolute value of the difference in the SP values of (A) and (B) is preferably 2.5 or less, and more preferably 1.5 or less. A colorless and transparent clear-type coating agent composition, pressure-sensitive adhesive composition, adhesive composition, ink composition, three-dimensional modeling ink composition, paint composition, sealing material composition, nail cosmetic, dental material, and decorative coating agent containing the curable composition (D), and colorless and transparent cured products and molded products thereof can be obtained, and can be suitably used as materials for optical members, electronic devices, and the like. When two or more polyfunctional (meth)acrylamide (A) and/or polymerizable compounds (B) are used in the curable composition, the SP value of A is an average value based on the mass ratios of all (A), and the SP value of B is an average value based on the mass ratios of all (B). In addition, when the structure of (A) or (B) contains a repeating unit, the SP value of (A) or (B) is an average value of the SP values of those repeating units.

One embodiment of the present disclosure is the various curable compositions (D) described above, wherein the acrylic equivalent of the polyfunctional (meth)acrylamide (A) is 180 or more. In this present disclosure acrylic equivalent is molecular weight per (meth)acrylamide, i.e., molecular weight divided by the number of acrylic groups. When the acrylic equivalent is low, the density of the(meth)acrylamide group is high and the curability is high, but the cure shrinkage resistance is low. When the acrylic equivalent of the polyfunctional (meth)acrylamide (A) is less than 180, the density of (meth)acrylamide group, which is a hydrophilic functional group in the molecule of (A), is too high, and (A) may be solid at normal temperature or may be water-soluble, which may cause problems such as difficulty in handling, cloudiness or unevenness of the obtained curable composition and cured product. The normal temperature in the present disclosure is a temperature range of 5 to 35 °C. The higher the acryl equivalent of the polyfunctional (meth)acrylamide (A) is, the easier it is to balance the hydrophilic property and the hydrophobic property, which is preferable. However, when it exceeds 3000, the molecular weight and the viscosity of (A) become high, and there is a risk of poor handling and curing properties. From these viewpoints, the acrylic equivalent of (A) is preferably 190 to 2500, and more preferably 200 to 2000.

One embodiment of the present disclosure is the various curable compositions (D) described above, wherein the polyfunctional (meth)acrylamide (A) is a compound represented by any one of general formulas [1] to [4].

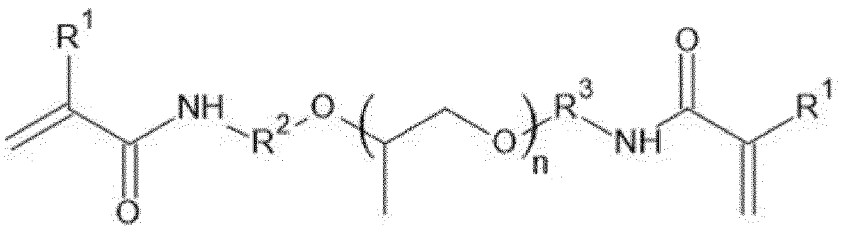

[Chemical formula 1]

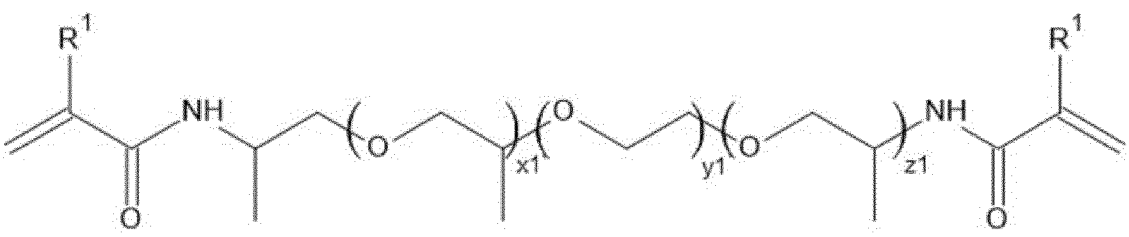

[Chemical formula 2]

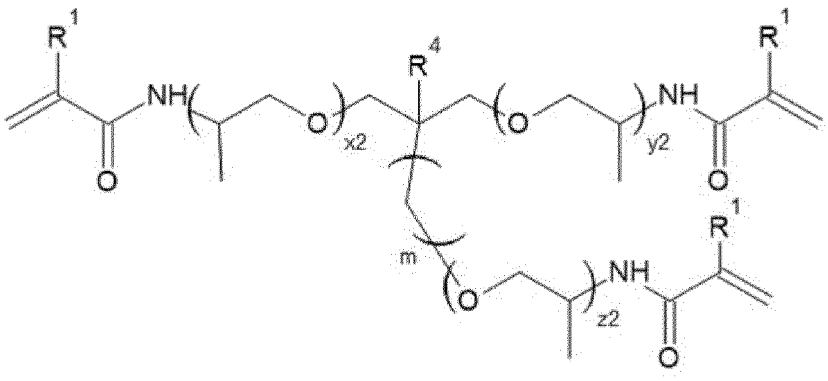

[Chemical formula 3]

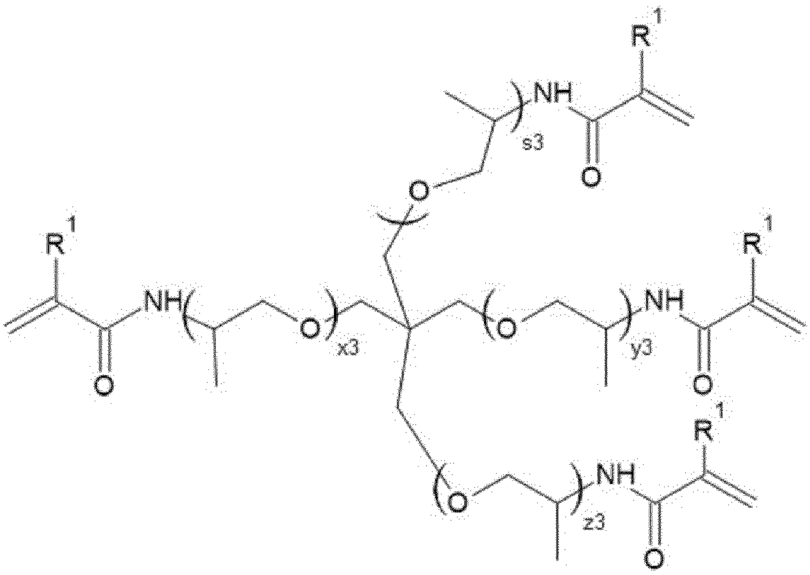

[Chemical formula 4]

In the general formulas [1] to [4], $R^1$ represents a hydrogen atom or a methyl group. $R^2$ and $R^3$ each represent a divalent chain hydrocarbon group having 3 carbon atoms, and may be linear or branched, and may be the same or different from each other. $R^4$ represents a hydrogen atom or a chain hydrocarbon group having 1 to 2 carbon atoms. n is an integer of 1 to 70, and m is 0 or 1. x1 and z1 each independently represent an integer of 1 to 10, y1 is an integer of 1 to 40, x2, y2, and z2 each independently represent an integer of 1 to 30, and s3, x3, y3 and z3 each independently represent an integer of 1 to 20.

Specific examples of the polyfunctional (meth)acrylamide (A) represented by the general formulas [1] to [4] include polypropyleneoxydi(meth)acrylamide, polypropyleneoxypolyethyleneoxydi(meth)acrylamide, trimethylolpropane polypropyleneoxytri(meth)acrylamide, pentaerythritolpolypropyleneoxytetra (meth)acrylamide, and the like. When the number of the repeating units of propyleneoxy groups or ethyleneoxy groups in these compounds is 1 to 30, the balance between the acrylic equivalent and the viscosity of (A) is easily adjusted. That is, the compound (A) has an appropriate acrylic equivalent, is liquid or wax-like at room temperature, and has good handleability, which is preferable. From these viewpoints, the number of the repeating unit is more preferably 1 to 20, and particularly preferably 2 to 10.

The polyfunctional (meth)acrylamide (A) is preferably a compound having a structure represented by any one of the general formulas [1] to [4]. When (A) has many ether groups in the molecule, (A) has the effect of suppressing the inhibition of photo-radical polymerization caused by oxygen. In addition, by having repeating units of isopropyleneoxy, (A) is hydrophilic while the cured product of the curable composition containing (A) can be obtained with sufficiently satisfactory water resistance. Furthermore, the polyfunctional (meth)acrylamide (A) represented by general formulas [1] to [4] are water-insoluble, it is possible to prepare a water-soluble or water-dispersible aqueous curable composition by combining (A) with a water-soluble curable compound (B). This is because (A) contains many hydrophilic (meth) acrylamide groups and is therefore easily compatibilized with the water-soluble (B) at the molecular level. Furthermore, the polyfunctional (meth)acrylamide (A) of the general formulas [3] and [4] are trifunctional and tetrafunctional, respectively, they have a branched structure, and therefore have a feature of low shrinkage upon curing, similarly to the bifunctional (A) of the general formulas [1] and [2].

One embodiment of the present disclosure is the various curable compositions (D) as described above, wherein the content of the polyfunctional (meth)acrylamide (A) is 1 to 95% by mass and the content of the polymerizable compound (B) is 5 to 99% by mass based on the total mass of the curable composition. When the contents of (A) and (B) are within these ranges, the obtained curable composition can satisfy all of properties and characteristics such as low viscosity, low cure shrinkage, high transparency, high curability, high wettability and adhesiveness, and at the same time, can be dissolved or dispersed in both organic solvents and water, and curable compositions (D) in various forms such as organic systems, aqueous systems and emulsions can be obtained. Although the polyfunctional (meth)acrylamide (A) is water-insoluble, it can be dissolved in water by coexisting with a water-soluble polymerizable compound (B), and an aqueous curable composition can be easily obtained. In the present disclosure, the term of "being soluble in water (water-soluble)" means that 1 g or more of the compound can be dissolved in 100 g of water at 25 °C to obtain a stable aqueous solution. In addition, the contents of (A) and (B) in (D) are preferably 2 to 90% by mass and 10 to 98% by mass, respectively, and more preferably 5 to 80% by mass and 20 to 95% by mass, respectively, from the viewpoint that the strength, hardness, heat resistance and the like of the cured product obtained by curing the curable composition (D) can be easily adjusted according to various applications.

One embodiment of the present disclosure is the various curable compositions (D) described above, wherein the polymerizable compound (B) has one or more polymerizable groups selected from the group consisting of (meth)acrylate groups, (meth)acrylamide groups, vinyl groups, vinyl ether groups, methyl vinyl ether groups, allyl groups, (meth)allyl ether groups, maleimide groups, α-substituted maleimide groups, and α, β-substituted maleimide groups. Note that (B) is a compound other than the polyfunctional (meth)acrylamide (A). The polymerizable group of the polymerizable compound (B) has ethylenically unsaturated bonds and can be polymerized by applying energy such as light or heat, and therefore can obtain a crosslinkable polymer (cured product) by polymerization reaction with the polyfunctional (meth)acrylamide (A). From the viewpoint of high polymerizability, the polymerizable group of (B) is preferably a (meth)acrylate group, a (meth)acrylamide group, or a vinyl group. From the viewpoint of having a self-photopolymerization initiation effect upon irradiation with active energy rays such as light, a vinyl ether group, a methyl vinyl ether group, a (meth)allyl ether group, a maleimide group, an α-substituted maleimide group, or an α,β-substituted maleimide group is preferable. Selection of the polymerizable group of the polymerizable compound (B) can be adjusted and designed according to preferable properties and characteristics of the curable composition (D) and a cured product obtained by curing the curable composition (D).

One embodiment of the present disclosure is the various curable compositions (D) described above, -wherein the polymerizable compound (B) contains a monofunctional polymerizable compound (b1) and/or a polyfunctional polymerizable compound (b2). Based on the total mass of the curable composition, the content of (b1) is 0 to 80% by mass and the content of (b2) is 0 to 40% by mass. By containing (b1) or (b2), a curable composition containing polyfunctional (meth) acrylamide (A) and polymerizable compound (B), which has high curability and transparency, and has good wettability and adhesion to various substrates, can be obtained. Further, by containing (b1) and (b2), a curable composition that is a liquid at normal temperature, has excellent handleability, and can be used in both aqueous system and organic system. From the viewpoint that the monofunctional polymerizable compound (b1) can reduce the viscosity of the curable composition (D) and improve the handleability, the content of (b1) is preferably 5% by mass or more, more preferably 10% by mass or more, and particularly preferably 20% by mass or more. On the other hand, when the content of (b1) exceeds 80% by mass, the total of the polyfunctional (meth)acrylamide (A) and the polyfunctional polymerizable compound (b2) in the curable composition (D) is less than 20% by mass, and depending on the application of (D), the surface hardness and strength of the obtained cured product may not be fully satisfied. From the viewpoint that the polyfunctional polymerizable compound (b2) can improve the curability of the curable composition (D), the content of (b2) is preferably 2% by mass or more, more preferably 5% by mass or more, and particularly preferably 10% by mass or more. On the other hand, when the content of (b2) exceeds 40% by mass, the total of the polyfunctional (meth)acrylamide (A) and the polyfunctional polymerizable compound (b2) in the curable composition (D) exceeds 41% by mass, the cure shrinkage of (D) increases, and there is a possibility that the obtained cured product is deformed or cracked.

The monofunctional polymerizable compound (b1) and the polyfunctional polymerizable compound (b2) have one or more polymerizable groups arbitrarily selected from the group of various polymerizable groups described above. From the viewpoint of high polymerizability, curability, and hydrophilicity, the polymerizable groups of (b1) and (b2) are preferably (meth)acrylate groups or (meth)acrylamide groups. Furthermore, in the water-based curable composition, it is particularly preferable to use water-soluble (b1) and/or (b2).

Examples of the monofunctional polymerizable compound (b1) as a monofunctional (meth)acrylate include methyl(meth)acrylate, ethyl(meth)acrylate, propyl(meth)acrylate, isopropyl(meth)acrylate, N-butyl(meth)acrylate, isobutyl(meth)acrylate, hydroxyethyl(meth)acrylate, tert-butyl(meth)acrylate, 2-ethylhexyl(meth)acrylate, isodecyl(meth)acrylate, lauryl(meth)acrylate, stearyl(meth)acrylate, isostearyl(meth)acrylate, tridecyl(meth)acrylate, methoxyethyl(meth) acrylate, ethoxyethyl(meth)acrylate, propoxyethyl(meth)acrylate, butoxyethyl(meth)acrylate, methoxydiethyleneglycol(meth)acrylate, methoxytriethyleneglycol(meth)acrylate, methoxytetraethyleneglycol(meth)acrylate, 2-(2-ethoxyethoxy)ethyl(meth)acrylate, phenoxyethyl(meth)acrylate, phenoxydiethyleneglycol(meth)acrylate, phenoxytetraethyleneglycol(meth)acrylate, phenoxyhexaethyleneglycol(meth)acrylate, methoxydipropyleneglycol(meth)acrylate, methoxytripropyleneglycol(meth)acrylate, cyclohexyl(meth)acrylate, tert-butylcyclohexyl(meth)acrylate, benzyl(meth)acrylate, phenoxyethyl(meth)acrylate, dicyclopentanyl(meth)acrylate, dicyclopentenyl(meth)acrylate, bornyl(meth)acrylate, isobornyl(meth)acrylate, tetrahydrofurfuryl(meth)acrylate, 2-methyl-2-adamantyl(meth)acrylate, allyl(meth)acrylate, hydroxyethyl(meth)acrylate, hydroxypropyl(meth)acrylate, hydroxybutyl(meth)acrylate, and hydroxyhexyl(meth)acrylate.

Examples of the monofunctional (meth)acrylamide of the monofunctional polymerizable compound (b1) include N-alkyl (saturated or unsaturated straight or branched chains having 1 to 18 carbon atoms) (meth)acrylamides such as N-methyl (meth)acrylamide, N-ethyl(meth)acrylamide, N-propyl(meth)acrylamide, N-isopropyl (meth)acrylamide, N-butyl(meth)acrylamide, N-isobutyl(meth)acrylamide, and N-hexyl(meth)acrylamide, N-alkoxy(straight or branched chain having 1 to 6 carbon atoms) alkyl (straight or branched chain having 1 to 6 carbon atoms) (meth)acrylamide such as N-methoxymethyl(meth)acrylamide, N-ethoxymethyl(meth)acrylamide, N-methoxyethyl(meth)acrylamide, N-ethoxyethyl(meth)acrylamide, N-n-butoxymethyl(meth)acrylamide, and hydroxyalkyl (straight or branched chain having 1 to 6 carbon atoms) (meth)acrylamides such as N-isobutoxymethyl(meth)acrylamide, N-vinylpyrrolidone, N-vinylcaprolactam, N-(2-hydroxyethyl)(meth)acrylamide, N-(2-hydroxypropyl)(meth)acrylamide, N-(3-hydroxypropyl)(meth)acrylamide, and N-(3-(dimethylamino))propyl(metha)acrylamide, N,N-dimethyl(meth)acrylamide, N,N-diethyl(meth)acrylamide, N,N-di-n-propyl(meth)acrylamide, N,N-diisopropyl(meth)acrylamide, N,N-di-n-butyl(meth)acrylamide, N,N-diisobutyl(meth)acrylamide, N-(meth)acryloylmorpholine, allyl(meth)acrylamide, 2-ethylhexyl(meth)acrylamide, and diacetoneacrylamide. These monofunctional polymerizable compounds (b1) may be used alone or in combination of two or more kinds thereof.

Examples of the polyfunctional polymerizable compound (b2) include monomers and oligomers of polyfunctional (meth) acrylates such as ethyleneglycoldi(meth)acrylate, diethyleneglycoldi(meth)acrylate, propyleneglycoldi(meth)acrylate, tripropyleneglycoldi(meth)acrylate, ditetraethyleneglycoldi(meth)acrylate, polyethyleneglycoldi(meth)acrylate, polypropyleneglycoldi(meth)acrylate, polytetramethyleneglycoldi(meth)acrylate, 1,3-butanedioldi(meth)acrylate, 1,4-butanedioldi(meth)acrylate, neopentylglycoldi(meth)acrylate, 1,6-hexanedioldi(meth)acrylate, 1,7-heptanedioldi(meth) acrylate, 1,8-octanedioldi(meth)acrylate, 1,9-nonanedioldi(meth)acrylate, hydroxypivalicacidneopentylglycoldi(meth) acrylate, dicyclopentanyldi(meth)acrylate, caprolactonemodifieddicyclopentenyldi(meth)acrylate, ethyleneoxidemodifiedphosphoricaciddi(meth)acrylate, pentaerythritoltetra(meth)acrylate, pentaerythritoltri(meth)acrylate, dipentaerythritolhexa(meth)acrylate, dipentaerythritolpenta(meth)acrylate, dipentaerythritoltetra(meth)acrylate, trimethylolethanetri(meth)acrylate, trimethylolpropanetri(meth)acrylate, tricyclodecanedimethanoldi(meth)acrylate, ethyleneoxidemodifiedbisphenolAdi(meth)acrylate, propyleneoxidemodifiedbisphenolAdi(meth)acrylate, cyclohexanedimethanoldi(meth) acrylate, acrylateester(dioxaneglycoldiacrylate), alkoxylatedhexanedioldi(meth)acrylate, alkoxylatedcyclohexanedimethanoldi(meth)acrylate, epoxy(meth)acrylate, andurethane(meth)acrylate.

Examples of the polyfunctional (meth)acrylamide for the polyfunctional polymerizable compound (b2) include methylenebis(meth)acrylamide, ethylenebis(meth)acrylamide, N,N'-(2-methylpropane-1,1-diyl)di(meth)acrylamide, N,N'-cyclohexylmethylenedi(meth)acrylamide, N,N'-(pentane-1,1-diyl) di(meth)acrylamide, N,N'-(3-methylbutane-1,1-diyl)di(meth)acrylamide, 2-(meth)acrylamido-2-methylpropanesulfonicacid, 1,4-bis(acryloyl)piperazine, ditrimethylolpropanetetra(meth)acrylamide, and dipentaerythritol hexa(meth)acrylamide. These polyfunctional polymerizable compounds (b2) may be used alone or in combination of two or more kinds thereof.

One embodiment of the present disclosure is the various curable compositions (D) described above, wherein the compositions (D) further contain a polymerizable polymerization initiator (C). The content of (C) is 0.1 to 20% by mass relative to the total mass of the curable composition. (C) is a compound other than the polyfunctional (meth)acrylamide (A) and the polymerizable compound (B), which has one or more polymerizable groups and one or more polymerization initiating functional groups (initiating groups)-in the molecule. The polymerizable group of (C) is one or more groups selected from the group consisting of a (meth)acrylate group, a (meth)acrylamide group, a vinyl group, a vinyl ether group, a methyl vinyl ether group, an allyl group, a (meth)allyl ether group, a maleimide group, an $\alpha$-substituted maleimide group, and an $\alpha,\beta$-substituted maleimide group. The initiating group of (C) is not particularly limited as long as it generates radicals, cations, anions or the like as a growing -active species by light irradiation or heating. For example, in the case of a curing reaction with an active energy ray such as UV or EB, examples of initiator group include intramolecular cleavage type initiators that generate radicals by intramolecular cleavage after absorbing light such as UV, hydrogen abstraction type initiators that generate radicals by exchange of hydrogen or electrons, and electron donating type initiators. More particularly, examples of the intramolecular cleavage type initiator group include benzoin derivative, benzyl ketal, $\alpha$-hydroxyacetophenone, $\alpha$-aminoacetophenone, acyl phosphineoxide, titanocenes and o-acyloximes. Examples of the hydrogen-abstracting type initiator group include benzophenone derivative having diarylketone skeleton such as benzophenone, alkyldiaminobenzophenone, 4, 4'-bis (dimethylamino)benzophenone and 4-benzoyl-4'-methyldiphenylsulfide, and thioxanthone derivative having thioxanthone skeleton such as 2-hydroxythioxanthone. One or more of these initiator groups can be selected and used in combination with one or more of the polymerizable groups. Among them, hydrogen abstracting type initiating groups are preferred due to no low molecular weight compound is produced as a by-product after the radical generation reaction and no low molecular weight compound remaining in the cured product. It is more preferable to have a structure in which a hydrogen abstracting type initiating group is combined with (meth)acrylate group or (meth)acrylamide group which is a polymerizable group with high polymerizability. Furthermore, in the aqueous curable composition, it is particularly preferable to use a water-soluble or hydrophilic polymerizable polymerization initiator (C).

The content of the polymerizable polymerization initiator (C) varies depending on the structure of the initiating group, the polymerizable group, the composition of the curable composition , and when the content is 0.1% by mass or more with respect to the total mass of the curable composition, thermal polymerization, photopolymerization, active energy ray curing, and the like can be immediately initiated, and the curable composition can be sufficiently cured. Furthermore, when (C) has a polymerizable group, if the content is 20% by mass or less, the curable composition can be rapidly cured, while the properties of cured products do not decrease. For adjusting the properties of the cured products suitably according to various applications, the content of the polymerizable polymerization initiator (C) with respect to the entire curable composition is preferably 0.5 to 15% by mass, and more preferably 1 to 10% by mass.

One embodiment of the present disclosure is the various curable compositions (D) further containing quaternary salt monomer. Quaternary salt monomer is a compound other than the polyfunctional (meth)acrylamide (A) and the polymerizable compound (B), and is a compound having both a polymerizable group and a quaternary structure in the molecule, and may be monofunctional or polyfunctional. The polymerizable group of quaternary salt monomer is one or more groups selected from the group consisting of (meth)acrylate group, (meth)acrylamide group, vinyl group, vinyl ether group, methyl vinyl ether group, allyl group, (meth)allyl ether group, maleimide group, $\alpha$-substituted maleimide group, and $\alpha,\beta$-substituted maleimide group. The quaternary salt monomer may be cationic, anionic, or amphoteric. The quaternary salt monomer may be a compound in which one or more cationic groups and/or anionic groups, selected from the group consisting of ammonium salts, imidazolium salts, choline salts, sulfonium salts, pyrazolium salts, oxazolium salts, pyridinium salts, pyrrolidinium salts, phosphonium salts, carboxylates, sulfonates, and phosphates, are combined with one or more groups selected from the various polymerizable groups described above. Among these, quaternary ammonium salt monomers are particularly preferable because they have antistatic properties and antimicrobial properties, and have an effect of promoting the dissolution or dispersion of pigments in the ink composition. These quaternary salt monomers may be used alone or in combination of two or more.

The content of the quaternary salt monomer is 0.1 to 30% by mass relative to the total mass of the curable composition (D). Quaternary salt monomers are either water-soluble or water-insoluble, both are highly hydrophilic. When the quaternary salt monomer is contained in an organic curable composition at 30% by mass or less, the water resistance of the obtaining cured products does not decrease, which is preferable. In an aqueous curable composition, when the content is 0.1% by mass or more, the water solubility or water dispersibility of the curable composition are significantly improved, which is preferable. From these viewpoints, the content of the quaternary salt monomer content in the curable composition is preferably 0.5 to 25% by mass, and more preferably 1 to 20% by mass.

One embodiment of the present disclosure provides a coating agent composition (hereinafter also referred to as a coating agent) containing the various curable compositions described above. The polyfunctional (meth)acrylamide (A) and the polymerizable compound (B), which are essential components of the coating agent composition, can be brought in from the curable composition (D) according to each embodiment described above, and can also be added when the coating composition is prepared as necessary. It is preferable that the content of (A) is 1 to 60% by mass and the content of (B) is 20 to 99% by mass with respect to the total mass of the coating agent composition. By combining the amphiphilic (A) with the hydrophilic or hydrophobic or amphiphilic (B), a hydrophilic or hydrophobic or amphiphilic coating agent composition can be obtained. Further, although (A) is water-insoluble, a water-soluble coating agent composition or a coating agent composition in an emulsion state which can be stably and uniformly dispersed in water can be obtained by combining (A) with water-soluble (B). Since A, B and their contents can be easily adjusted, the obtaining coating composition has excellent compatibility with a wide range of compounds, from hydrophobic to water-soluble, other than A and B, and has high wettability with various materials, from organic to inorganic. The cured film (coating film) obtained by curing such a coating composition is uniform and has high transparency and surface smoothness without unevenness. In addition, (A) has many isopropyleneoxy groups, water resistance can be imparted to the obtaining cured film, and the appearance and surface hardness of the cured film can also be adjusted according to the application. For example, when A is used in combination with a monofunctional polymerizable compound (b1) as B, the surface of the cured film is not uneven, and the appearance such as surface smoothness is improved. When A is used in combination with a polyfunctional polymerizable compound (b2) as (B), the surface hardness of the cured film is significantly improved. These coating compositions can be suitably used for various coating applications such as hard coats, vehicles, and indoor or outdoor building materials.

One embodiment of the present disclosure provides a pressure-sensitive adhesive composition (hereinafter also referred to as a pressure-sensitive adhesive) containing any of the curable compositions described above. The polyfunctional (meth)acrylamide (A) and the polymerizable compound (B), which are essential components of the pressure-sensitive adhesive composition, can be brought in from the curable composition (D) according to each embodiment

described above, and can also be added when the pressure-sensitive adhesive is prepared as necessary. It is preferable that the content of (A) is 2 to 50% by mass and the content of (B) is 10 to 90% by mass with respect to the total mass of the pressure-sensitive adhesive composition. (A) is amphiphilic and has multiple (meth)acrylamide groups, and the interaction between the wettability due to the amphiphilicity and the cohesiveness of the (meth)acrylamide groups, gives the pressure-sensitive adhesive composition excellent adhesion to various substrates (from organic materials to inorganic materials) and stain resistance (reworkability that allows for removability). In addition, (A) is amphiphilic and has many (meth)acrylamide groups with high hydrolysis resistance and isopropyleneoxy groups with high water resistance and cure shrinkage resistance, and by the combination of (A) and (B), and the adhesive layer or adhesive sheet obtained by curing the pressure-sensitive adhesive composition by combining (A) and (B), and the laminate obtained by laminating through the adhesive layer have good yellowing resistance (including transparency) and durability (moist-heat resistance). In addition, the pressure-sensitive adhesive composition of the present embodiment can further contain polymerization initiators such as polymerizable polymerization initiators, non-polymerizable polymerization initiators, photopolymerization initiators, and thermal polymerization initiators, various solvents with low-polarity to high-polarity, general-purpose monomers, and various additives, and can be adjusted from low viscosity to high viscosity according to the application. Such a pressure-sensitive adhesive composition can also be suitably used in the optical field, such as a pressure-sensitive adhesive for optical members, a pressure-sensitive adhesive layer, and a pressure-sensitive adhesive sheet, etc. In addition, the obtained pressure-sensitive adhesive layer and laminates comprising of the pressure-sensitive adhesive layer and various substrates can be applied as pressure-sensitive adhesive films or pressure-sensitive adhesive sheets for electronic materials, optical members, and automobile members.

The pressure-sensitive adhesive composition of the present embodiment can form a pressure-sensitive adhesive layer by being cured by irradiation with an active energy ray after being applied to or molded on a separator or a substrate. When an organic solvent is contained in the pressure-sensitive adhesive composition, the composition may be coated or formed on a separator or a substrate and cured while evaporating (drying) the organic solvent by irradiation with active energy. It is preferable to heat and dry at a temperature of 60 to 120 °C for 1 to 30 minutes, and then cure with active energy, since this gives a more transparent adhesive layer The pressure-sensitive adhesive composition can be applied by a conventional coating method such as spin coating method, spray coating method, knife coating method, dipping method, gravure roll method, reverse roll method, screen-printing method, or a bar coater method.

A laminate can be obtained by laminating the pressure-sensitive adhesive layer obtained from the pressure-sensitive adhesive composition on various substrates. Examples of the lamination method include a transfer method and a roll-to-roll method. The thicknesses of the pressure-sensitive adhesive layers in the laminate vary depending on various applications and are therefore not particularly limited, but are usually 4 to 150 μm, and are suitably about 20 to 120 μm when used for automobile members and about 30 to 100 μm when used for electronic materials or optical members.

The pressure-sensitive adhesive composition of the present embodiment or the pressure-sensitive adhesive layer obtained therefrom exhibits high adhesion to various substrates, as a substrate to be adhered or a substrate to be laminated, a wide variety of substrates including low-polarity substrates to high-polarity substrates, organic substrate, inorganic substrate, and substrates made of organic-inorganic composite materials can be used. Examples include polyolefin resins such as polyethylene and polypropylene, polyester resins such as polyethylene terephthalate and polycarbonate, ABS resins which are acrylonitrile-butadiene-styrene copolymers, acrylic resins such as polyimide resins, polyamide resins and polymethyl methacrylate, metals such as steel, stainless steel, copper and aluminum, glass, and hybrid materials in which inorganic material silica particles are dispersed in organic material polyimide. The applications of various laminates obtained are not particularly limited, and examples thereof include electronic materials, materials for optical components and automotive parts.

One embodiment of the present disclosure provides an adhesive composition (hereinafter also referred to as an adhesive) containing any of the curable compositions described above. The polyfunctional (meth)acrylamide (A) and the polymerizable compound (B), which are essential components of the adhesive composition, can be brought in from the curable composition (D) according to each embodiment described above, and can also be added when the adhesive composition is prepared as necessary. It is preferable that the content of (A) is 2 to 70% by mass and the content of B is 20 to 90% by mass with respect to the total mass of the adhesive composition. (A) is amphiphilic and has multiple (meth) acrylamide groups. Due to the interaction between the wettability derived from the amphiphilicity and the cohesiveness of the (meth)acrylamide groups, the adhesive composition can adhere evenly and without unevenness to various substrates (from organic materials to inorganic materials), has high adhesive strength after being cured, and is suitably used for adhesion of the same kind of material and adhesion of different kinds of materials. Further, (A) has many of isopropyleneoxy groups and (meth)acrylamide groups, and the cured product (adhesive layer, adhesive laminate, etc.) of the adhesive composition containing (A) has good hydrolysis resistance and water resistance. When (A) is used in combination with the monofunctional polymerizable compound (b 1), the viscosity of the adhesive composition can be easily adjusted, and thin to thick adhesive layers can be produced depending on the application. When (A) is used in combination with the polyfunctional polymerizable compound (b2), the heat resistance and thermal shock resistance (cold and thermal impact resistance) of the cured product can be significantly improved. Furthermore, an adhesive composition

containing amphiphilic (A), various (B), a polymerizable or non-polymerizable polymerization initiator and the like is high transparency, and the adhesive layer obtained by curing the composition also maintains high transparency, and therefore such an adhesive composition can also be suitably used in the optical field such as adhesive for optical components.

One embodiment of the present disclosure provides an ink composition (hereinafter also referred to as an ink) containing any of the curable compositions described above. The polyfunctional (meth)acrylamide (A) and the polymerizable compound (B), which are essential constituent components of the ink composition, can be brought in from the curable composition (D) according to each embodiment described above, and can also be added when the ink composition is prepared as necessary. It is preferable that the content of (A) is 3 to 50% by mass and the content of (B) is 20 to 85% by mass with respect to the total mass of the ink composition. (A) is amphiphilic, and by mixing with (B) of various polarities from hydrophilic to hydrophobic, the dispersibility of various inorganic pigments and organic pigments is excellent, and an excellent coating property to various printing substrates is exhibited, and the print definition of the obtained printed matter is excellent. The ink composition has high curability by containing (A), and the obtaining printed face has good surface drying properties. Furthermore, by using A in combination with a polyfunctional polymerizable compound (b2), the surface drying properties of the printed surface obtained from the ink composition are further improved. By using A in combination with a monofunctional polymerizable compound (b1), an ink composition with a range of viscosities from low to high can be obtained. The ink composition of this embodiment can be suitably adjusted in viscosity depending on various applications and printing methods, and has high ejection stability during printing and high printing accuracy (including print clarity). The viscosity of the ink composition at 25 °C is preferably 1000 mPa·s or less, more preferably 500 mPa·s or less, and particularly preferably 100 mPa·s or less from the viewpoint of suitable use in ink jet method. After the ink composition is printed on a substrate, it is cured by irradiation with active energy rays or heat to form a printed surface (ink layer or printed layer). Furthermore, since quaternary salt monomer has excellent compatibility with pigments, the dissolution and dispersion of the pigment is promoted by including the quaternary salt monomer in the ink composition. From the viewpoint of achieving a good balance of printing characteristics such as the viscosity, pigment dispersibility, curability, and ejection stability of the ink composition, and the surface dryness and clarity of the resulting printed surface, the ink composition preferably contains 3 to 50% by mass of (A), 30 to 80% by mass of (b1), and 5 to 40% by mass of (b2). Such ink compositions can be suitably used in various printing methods such as inkjet printing, offset printing, screen printing, and flexographic printing. For example, offset printing is a printing method that utilizes the water-repellent properties of an oil-based printing ink composition, and by containing (A) and hydrophobic (B), the ink composition exhibits high hydrophobicity and curing properties, making it possible to achieve high-speed, high-precision printing. Furthermore, even in an ink composition containing (A) having many isopropyleneoxy groups and hydrophilic (B), the water resistance of the obtaining print is fully satisfactory.

One embodiment of the present disclosure provides an aqueous ink composition (hereinafter also referred to as an aqueous ink) containing any of the curable compositions described above. The polyfunctional (meth)acrylamide (A) and the polymerizable compound (B), which are essential components of the aqueous ink composition, can be brought in from the curable composition (D) according to each embodiment described above, and can also be added when the aqueous ink composition is prepared as necessary. It is preferable that the content of (A) is 1 to 60% by mass, the content of (B) is 5 to 89% by mass, and the content of water is 10 to 50% by mass with respect to the total mass of the aqueous ink composition. (A) is water-insoluble while is amphiphilic, it has good compatibility with the water-soluble (B), and the curable composition obtained by mixing with the water-soluble (B) can be used as an aqueous ink composition. Such an ink composition exhibits excellent solubility in water or excellent compatibility with water, exhibits good solubility or dispersibility in water-soluble pigment, etc., and has high storage stability as a pigment-free clear ink, and has high pigment dispersibility and storage stability as a pigment-containing ink. In addition, since the aqueous ink composition contains (A), it has high curability, and the obtaining printed surface is excellent in water resistance. (A) and (B) are highly compatible, and (D), which contains (A) and (B), is highly compatible with water or with additives such as water-soluble pigments. By containing (A) and (B), a wide variety of ink compositions can be prepared according to the purpose, such as ink compositions having a viscosity range applicable to inkjet printing. Furthermore, the ink composition has good ejection stability during inkjet printing, and excellent printing characteristics can be achieved. When the quaternary salt monomer is contained at 0.1% by mass or more, the solubility of A in water is significantly improved, and various properties of the aqueous ink composition and printed products using the same are further improved, which is preferable. The aqueous ink composition of this embodiment may be an aqueous solution that dissolves in water, or an aqueous dispersion such as an emulsion that disperses in water. The water content is 10% by mass or more, preferably 20% by mass or more, and more preferably 30% by mass or more, with respect to the total mass of the aqueous ink composition. The water is preferably ion-exchanged water, distilled water, or the like, that does not contain ionic impurities.

One embodiment of the present disclosure is an ink composition for three-dimensional modeling (hereinafter also referred to as an ink composition for modeling or an ink for modeling) containing the various curable compositions described above. The polyfunctional (meth)acrylamide (A) and polymerizable compound (B), which are essential components of the ink composition for modeling, can be brought in from the curable composition (D) according to each embodiment described above, and can also be added when the ink composition for modeling is prepared as necessary. It is

preferable that the content of (A) is 1 to 60% by mass and the content of (B) is 20 to 99% by mass with respect to the total mass of the modeling ink composition. (A) has multiple (meth)acrylamide groups and isopropyleneoxy groups, and the ink composition for modeling has high curing properties, and the obtaining modeled object (cured object) has good strength, toughness (impact resistance), and water resistance. By including a monofunctional polymerizable compound (b1) as (B), the viscosity of the ink composition for modeling can be suitably adjusted according to the device used for modeling, and the handleability of the ink composition for modeling is also improved. By including a polyfunctional polymerizable compound (b2) as (B), the curability of the ink composition for modeling and the strength, hardness, etc. of the obtained modeled object can be further improved. When the absolute value of the difference in SP values between (A) and (B) is 3.0 or less, (A) and (B) are highly compatible, and the ink composition for modeling has high transparency and high ink ejection stability during modeling. In addition, when the acrylic equivalent of (A) is 180 or more, the cure shrinkage resistance of the ink composition for modeling is higher, and a modeled object with excellent modeling accuracy can be obtained.

The ink composition for modeling is cured by irradiation with active energy rays or heat at the same time as or immediately after being formed into a predetermined shape pattern to form a thin film, and a three-dimensional object can be obtained by laminating the thin films. The modeling method is not particularly limited, and an example is a photo-modeling method in which the ink is ejected by an inkjet method and cured by exposure to active energy rays. In this case, from the viewpoint of ejection stability, the viscosity of the ink composition for modeling at 25°C is preferably 1 to 200 mPa·s, and the ejection temperature is preferably in the range of 20 to 100 °C. From the viewpoint that the obtaining three-dimensional model can exhibit a good balance of strength, modeling accuracy and impact resistance, it is more preferable that the ink composition for modeling contains 5 to 60% by mass of (A), 10 to 70% by mass of (b)1 and 5 to 50% by mass of (b)2.

One embodiment of the present disclosure is an aqueous coating composition (hereinafter also referred to as an aqueous coating) containing the various curable compositions described above. The polyfunctional (meth)acrylamide (A) and polymerizable compound (B), which are essential components of the aqueous coating composition, can be brought in from the curable composition (D) according to each embodiment described above, and can be added when the aqueous coating composition is prepared as necessary. It is preferable that the content of (A) is 1 to 50% by mass, the content of (B) is 5 to 79% by mass, and the content of water is 20 to 80% by mass, relative to the total mass of the aqueous coating composition. (A) is water-insoluble while amphipathic, and therefore has good compatibility with water-soluble (B). The curable composition obtained by mixing (A) with water-soluble (B) can be used as an aqueous coating composition. Such a coating composition exhibits excellent solubility or compatibility with water, and also exhibits good solubility or dispersibility in water-soluble pigments, etc., and has high storage stability as a pigment-free clear ink, and has high pigment dispersibility and storage stability as a pigment-containing ink. In addition, since the aqueous coating composition contains (A), it has high curability, and the obtaining coating film has excellent water resistance. Since (A) and (B) are highly compatible, and (D), which contains (A) and (B), is highly compatible with water or with additives such as water-soluble pigments, and since (A) has multiple (meth)acrylamide groups and isopropyleneoxy groups, the aqueous coating composition has high wettability and adhesion to various substrates such as wood, metal, concrete, rubber, ceramics, plastics, paper, fiber, and nonwoven fabric, and it can be used for coating multi-materials. Furthermore, when the quaternary salt monomer is contained at 0.1% by mass or more, the solubility of (A) in water is significantly improved, and various properties of the aqueous coating composition and coating films which is the cured product thereof are further improved, which is preferable. The aqueous coating composition of this embodiment may be an aqueous solution that dissolves in water, or an aqueous dispersion such as an emulsion that disperses in water. The water content is 20% by mass or more, preferably 30% by mass or more, and more preferably 50% by mass or more, with respect to the total mass of the aqueous coating composition. The water is preferably ion-exchanged water, distilled water, or the like, that does not contain ionic impurities.

The aqueous coating composition of the present embodiment can contain an organic solvent as necessary, and the content the organic solvent is 40% by mass or less, preferably 30% by mass or less, and more preferably 20% by mass or less with respect to the total mass of the coating components (the total of the organic solvent and water). After the aqueous coating composition is applied to a separator or a substrate to form a coating film, the coating film may be cured by irradiation with active energy such as UV or EB while evaporating (drying) the organic solvent and water. It is preferable to heat the coating film at a temperature of 60 to 120°C for 1 to 30 minutes for drying and then cure it with active energy rays, since this results in a coating film with higher transparency. The aqueous coating composition may be applied by a general coating method such as spin coating, spray coating, knife coating, dipping, dip coating, gravure roll coating, reverse roll coating, screen-printing or bar coater coating, and can also be suitably applied by manual coating using a roller brush, a brush, a spatula, or the like.

One embodiment of the present disclosure provides a sealant composition (hereinafter also referred to as a sealant) containing the various curable compositions described above. The polyfunctional (meth)acrylamide (A) and the polymerizable compound (B), which are essential components of the sealant composition, can be brought in from the curable composition (D) according to each embodiment described above, and can be added when the sealant composition is prepared as necessary. It is preferable that the content of (A) is 1 to 60% by mass and the content of (B) is 10 to 99% by

mass with respect to the total mass of the sealant composition. The (meth)acrylamide of (A) has high curability to both active energy rays and heat, and the sealant composition obtained by using (A) and (B) in combination also exhibits high curability. In addition, since (A) is polyfunctional, the amount of uncured polymerizable compound (residual monomer) in a cured product such as a sealing layer or a sealing agent obtained by curing the sealant composition is extremely small, and the cured product has high outgassing resistance, moist-heat yellowing resistance, corrosion resistance, and the like, and can exhibit an excellent sealing effect as a sealant. The compatibility between (A) and (B) is high, and the sealant composition and the cured products such as a sealing layer obtained by curing the sealant composition have good transparency and are also suitably used for optical applications. (A) has many isopropyleneoxy groups, and the cured product obtained by curing the sealant composition has the effects of water resistance and cure shrinkage resistance. Furthermore, the handleability of the sealant composition is improved by the combined use of (A) and the monofunctional polymerizable compound (b1), and the curability of the sealant composition and the strength and heat-sealing resistance of the obtained cured product are improved by the combined use of (A) and the polyfunctional polymerizable compound (b2).

One embodiment of the present disclosure provides a nail cosmetic composition (hereinafter also referred to as a nail cosmetic) containing the various curable compositions described above. The polyfunctional (meth)acrylamide (A) and the polymerizable compound (B), which are essential components of the nail cosmetic composition, can be brought in from the curable composition (D) according to each embodiment described above, and can be added when the nail cosmetic composition is prepared, as necessary. It is preferable that the content of (A) is 1 to 40% by mass and the content of (B) is 10 to 99% by mass with respect to the total mass of the nail cosmetic composition. (A) has multiple (meth)acrylamide groups, and the obtained nail cosmetic composition has high curability and high adhesiveness to nails, and the strength of the obtained cured product (also called cured film or nail) is also high. (A) and (B) are highly compatible, and the nail cosmetic composition and its cured film that do not contain pigment have high transparency and surface gloss. It has high compatibility with (A), (B) and other components such as pigments, and the nail cosmetic composition containing the pigment has high pigment dispersibility (uniformity), the obtaining cured film has high surface gloss, and there is almost no unevenness in the cured film due to recoating or repairing work. Furthermore, when the acrylic equivalent of the (A) is 180 or more, the nail cosmetic composition is more excellent in cure shrinkage resistance, no unevenness is generated on the surfaces of the cured films, and the appearance of the nails is good. By appropriately adjusting the (A), the monofunctional polymerizable compound (b1) and/or the polyfunctional polymerizable compound (b2) and the contents of them, it is possible to obtain a nail cosmetic composition that can be used for various substrates such as natural nails, artificial nails, nail films, and nail chips.

One embodiment of the present disclosure provides a dental material composition (hereinafter also referred to as a dental material) containing the various curable compositions described above. The polyfunctional (meth)acrylamide (A) and the polymerizable compound (B), which are essential components of the dental composition, can be brought in from the curable composition (D) according to each embodiment described above, or can be added when the dental composition is prepared as necessary. It is preferable that the content of (A) is 0.5 to 50% by mass and the content of (B) is 5 to 99% by mass with respect to the total mass of the dental material composition. (A) has multiple (meth)acrylamide groups, and the dental material composition has high curability and adhesion, and when used as an adhesive dental material, high adhesive strength can be obtained. In addition, when the acrylic equivalent of (A) is 180 or more, the dental material composition has high cure shrinkage resistance, does not show deformation with time after curing or surface unevenness, and has good surface smoothness. The compatibility between (A) and (B) is high, the dispersibility of white pigments and inorganic fillers is excellent, and they are suitably used as composite resins, fillings and coverings for the treatment and restoration of natural teeth, and as dental materials used for the preparation of artificial teeth such as artificial teeth, bridges and implants. Furthermore, the viscosity of the dental material composition can be easily adjusted, and the handleability can be improved by using (A) in combination with the monofunctional polymerizable compound (b1). When (A) is used in combination with the polyfunctional polymerizable compound (b2), the dental material composition has higher curability and the obtaining cured product has higher hardness.

One embodiment of the present disclosure provides a decorative coating agent composition (hereinafter, also referred to as a decorative coating agent) containing the various curable compositions described above. The polyfunctional (meth)acrylamide (A) and the polymerizable compound (B), which are essential components of the decorative coating agent composition, can be brought in from the curable composition (D) according to each embodiment described above, and can be added when the decorative coating agent composition is prepared as necessary. It is preferable that the content of (A) is 1 to 50% by mass and the content of (B) is 20 to 99% by mass with respect to the total mass of the decorative coating agent composition. multiple (meth)acrylamide groups, and the obtained decorative coating agent composition has high curability, and the obtained cured product such as a decorative coating, a decorative film, or a decorative film has good surface hardness and scratch resistance. In addition, (A) has many isopropyleneoxy groups, and the obtained cured product exhibits flexibility, and the decorative coat, the decorative film, and the decorative film are excellent in bending resistance. (A) is amphiphilic and has high compatibility with (B), and an oligomer, a polymer or the like can be further dissolved in (D) containing (A) and (B), and the cured product obtained from such a composition has a higher elongation.

In each embodiment of the present disclosure, the curable composition (D) may contain a component other than those described above as an optional component as necessary. As optional components, an organic solvent and various additives can be used as required. Examples of the organic solvents include alcohols such as ethyl alcohol, N-propyl alcohol, and isopropyl alcohol; ketones such as acetone, methylethylketone, and methyl isobutyl ketone; alkylene glycols; polyalkyleneglycols; aromatic hydrocarbons such as glycol ethers, glycol esters, benzene, toluene, ethylbenzene, and xylene; aliphatic hydrocarbons such as hexane, heptane, octane, decade, and cyclohexane; esters such as ethyl acetate, butyl acetate, and aceticacid-2-hydroxyethyl; acetonitrile; and N,N-dimethylformamide. These organic solvents may be used alone or in combination of two or more. In particular, in the case of an organic curable composition, ethyl acetate, methylethylketone, acetone, or the like having a low boiling point is more preferable from the viewpoint of easy removal by a method such as drying during coating, film formation, or modeling or after modeling, and in the case of an aqueous curable composition, ethyl alcohol, N-propyl alcohol, isopropyl alcohol, or the like, which are water-soluble and have a low boiling point, are more preferable. When the organic solvent is contained, the content thereof is preferably 1.0 to 50% by mass, and more preferably 5 to 30% by mass, with respect to the total mass of the curable composition. Within this range, there is no risk of adversely affecting the properties expected for the composition of the embodiment of the present disclosure and the cured product obtained by curing them.

Examples of the additives used in the present disclosure include polymerization initiators (excluding the polymerizable polymerization initiator (C)), thermal polymerization inhibitors, anti-aging agents, antioxidants, ultraviolet sensitizers, preservatives, phosphate and other flame retardants, surfactants, wetting dispersants, antistatic agents, colorants, plasticizers, surface lubricants, leveling agents, softeners, pigments, organic fillers, inorganic fillers, and silica particles. The addition amount of these additives is not particularly limited as long as expected characteristics of the composition of each embodiment of the present disclosure and a cured product obtained by curing the composition are not adversely affected, and is preferably 5% by mass or less and more preferably 2% by mass or less with respect to the total mass of the curable composition.

In each embodiment of the present disclosure, a non-polymerizable component can be further contained as necessary. The non-polymerizable component is a compound having no polymerizable group in the molecule. When non-polymerizable components are classified by molecular weight (weight-average molecular weight (Mw)), a non-polymerizable component having a Mw of 1,000 or more and less than 10,000 is regarded as a non-polymerizable oligomer, and a non-polymerizable component having a Mw of 10,000 or more is regarded as a non-polymerizable polymer. Examples of the non-polymerizable oligomer and the non-polymerizable polymer include a thermoplastic resin, a rosin-based resin, and a mixture thereof. Examples of the thermo-plastic resins include (meth)acrylic resin; polycycloolefin resin; cellulose resin; polyester resin; polyurethane resin; polysulfonic acid resin; copolymers of acrylonitrile with butadiene and styrene; polycarbonate resin; polyamide resin and polyimide resin. Examples of the rosin-based resins include natural rosins such as gum rosin, and modified rosin resin such as hydrogenated rosins, disproportionated rosins, rosin-modified phenolic resin, maleic acid modified rosin resin, maleated rosins, and esterified gums obtained by modifying natural rosins. These non-polymerizable oligomers and non-polymerizable polymers may be used alone or in combination of two or more thereof.

The content of the non-polymerizable component is preferably 0.1 to 20% by mass with respect to the total mass of the curable composition. The non-polymerizable component has an effect of adjusting the viscosity of the curable composition, an effect of improving the adhesiveness of the pressure-sensitive adhesive composition and the adhesive composition to the base material, an effect of improving the adhesive force of the pressure-sensitive adhesive layer and the adhesive force of the adhesive, and an effect of imparting toughness to the coating agent composition, the ink composition, and the cured product of the three dimensional modeling ink composition, the sealant composition, and the decorative coating agent composition. From these viewpoints, the content of the non-polymerizable component is more preferably 0.5 to 15% by mass, and particularly preferably 1 to 10% by mass.

The curable composition according to each embodiment of the present disclosure can be cured by an active energy ray such as UV or EB and/or heat, and a cured product suitably used for various applications can be obtained. For example, the pressure-sensitive adhesive layer can be formed by applying a curable composition as the pressure-sensitive adhesive composition to a separator or various substrates and then curing the composition with an active energy ray. In the present disclosure, curing the curable composition with an active energy ray and heat is also referred to as hybrid curing. In hybrid curing, active energy rays and heat may be applied in this order, or heat and active energy rays may be applied in this order, or thermal curing utilizing the amount of heat generated by active energy ray curing, that is, simultaneous curing with active energy rays and heat may be performed.

The active energy ray described above is defined as an energy ray capable of decomposing a compound capable of generating an active species (photopolymerization initiator) to generate an active species. Examples of such an active energy ray include visible light, ultraviolet, infrared ray $\alpha$-ray, $\beta$-ray, $\gamma$-ray, x-ray and an electronic beam (EB). When an electron beam is used as the active energy ray, a photopolymerization initiator may not be used. On the other hand, when ultraviolet light, visible light, or the like is used, a photopolymerization initiator is preferably used. The irradiation with an active energy ray is preferably performed in an atmosphere of an inert gas such as nitrogen gas or carbon dioxide gas, or in

an atmosphere with a reduced oxygen concentration. The curable composition according to each embodiment of the present disclosure has good curability due to having the polyfunctional (meth)acrylamide (A), and can be sufficiently cured even in a normal air atmosphere. The irradiation temperature of the active energy rays is preferably 10 to 200 °C, and the irradiation time is preferably 1 second to 60 minutes.

Non-polymerizable photopolymerization initiator may be a substance (that is, a photo-radical polymerization initiator) which generates a radical by irradiation with ultraviolet rays having an appropriate wavelength capable of causing a polymerization reaction depending on the kind of the active energy ray reactive component. Such photopolymerization initiators may be appropriately selected from acetophenone-based, benzoin-based, benzophenone-based, thioxanthone-based, and the like. Commercially available products including Omnirad 1116, Omnirad 1173, Omnirad 184, Omnirad 369, Omnirad 500, Omnirad 651, Omnirad 754, Omnirad 819, Omnirad 907, Omnirad 1300, Omnirad 1800, Omnirad 1870, Omnirad 2959, Omnirad 4265, Omnirad TPO, Omnipol 2702, Omnipol 910, Omnipol 9210, Omnipol 2702, Omnipol BP, Omnipol TP, Omnipol TX and the like from IGM Resins B.V., Speed Cure 7005, Speed Cure 7010, and the like from Arkema, FOM-03011 and the like from Fujifilm Wako Pure Chemical Industries, Ltd., Ubecryl P36 and the like from UCB can be used. These non-polymerizable photopolymerization initiators may be used alone or in combination of two or more kinds thereof.

The non-polymerizable photo-radical polymerization initiator is not particularly limited, and examples thereof include benzoin-based such as benzoin, benzoin methyl ether, benzoin ethyl ether, benzoin isopropyl ether, benzoin isobutyl ether, anisole methyl ether; acetophenones-based such as 4-(2-hydroxyethoxy)phenyl(2-hydroxy-2-propyl) ketone, α-hydroxy-α, α'-dimethyl acetophenone, methoxy acetophenone, 2,2'-dimethoxy-2-phenyl acetophenone, 2-hydroxy-2-cyclohexyl acetophenone, 2,2-diethoxy acetophenone, 2,2-dimethoxy-2-phenyl acetophenone, 1-hydroxycyclohexylphenyl ketone, 4-phenoxy dichloroacetophenone and 4-tert-butyl-dichloroacetophenone; benzophenone-based such as 2-hydroxy-2-methyl propiophenone, propiophenone such as 2-hydroxy-4'-isopropyl-2-methyl propiophenone, benzophenone, methylbenzophenone, p-chlorobenzophenone, and p-dimethylaminobenzofuranone; thioxanthone-based such as thioxanthone, 2-chlorothioxanthone, 2-chillthioxanthone, 2-isopropylthioxanthone, 2,4-dichlorothioxanthone, 2,4-diethylthioxanthone, 2,4-diisopropylthioxanthone and dodecyl thioxanthone; acylphosphine oxides such as bis(2,4,6-trimethylbenzoyl)-phenylphosphine oxide, 2,4,6-trimethylbenzoyldiphenylphosphine oxide, (bis(2,4,6-trimethylbenzoyl)-2,4-di-n-butoxyphenylphosphine oxide, bis(2,6-dimethoxybenzoyl)-2,4,4-trimethylpentylphenylphosphine oxide), benzyl, dibenzosuberone, and α-acyloxime ester, etc. These non-polymerizable photo-radical polymerization initiators may be used alone or in combination of two or more kinds thereof.

The content of the non-polymerizable photopolymerization initiator is 0.1 to 20% by mass, preferably 0.5 to 10% by mass, and more preferably 1 to 5% by mass with respect to the total mass of the curable composition according to each embodiment of the present disclosure. If the content of the non-polymerizable photopolymerization initiator is less than 0.1 % by mass, sufficient curability may not be obtained, and if it exceeds 20% by mass, performance such as strength of the cured product may decrease. In addition, the polymerizable photopolymerization initiator (C) and the non-polymerizable photopolymerization initiator may be used in combination.

Thermal curing of the curable composition according to each embodiment of the present disclosure can be carried out by a known method in the presence of a thermal polymerization initiator. For example, methods such as emulsion polymerization, solution polymerization, suspension polymerization, or bulk polymerization can be used, and a crosslinked cured product can be obtained. When using the solution polymerization method, examples of solvents that can be used include aromatic hydrocarbons such as toluene, ethylbenzene, and xylene; aliphatic hydrocarbons such as hexane, heptane, octane, decane, and cyclohexane; esters such as ethyl acetate, butyl acetate, and 2-hydroxyethyl acetate; aliphatic alcohols such as ethyl alcohol, n-propyl alcohol, and isopropyl alcohol; ketones such as acetone, methyl ethyl ketone, and methyl isobutyl ketone; acetonitrile; and N,N-dimethylformamide. These solvents may be used alone or in combination of two or more. In particular, from the viewpoint of easy removal from the cured product, it is preferable to use low-boiling point ethyl acetate, methyl ethyl ketone, acetone, etc. The temperature and time of thermal polymerization vary depending on the thermal polymerization method adopted and the thermal polymerization initiator used but are usually calculated from the half-life of the initiator. The temperature is usually preferably 60 to 120 °C, and the time is usually preferably 2 hours to 20 hours, and more preferably 5 hours to 10 hours.

The thermal polymerization initiator may be a thermal radical polymerization initiator. For example, azo-based initiators such as azobisisobutyronitrile, azobisvaleronitrile, azobis(isobutyrate)dimethyl, tetrahydrate of 2,2'-azobis(N-(2-carboxyethyl)-2-methylpropionamidine), 2'-azobis(2-methyl-N-(2-hydroxyethyl)propionamide), 2,2'-azobis(2-(2-imidazolin-2-yl)propane); benzoyl peroxide-based initiators; peroxide-based initiators such as hydrogen peroxide; and persulfate-based initiators such as ammonium persulfate and sodium persulfate may be used. The content of the thermal polymerization initiator is about 0.01 to 10% by mass relative to the total mass of the curable composition. Furthermore, ordinary radical polymerization techniques such as adjusting of molecular weight using a chain transfer agent can be applied.

EXAMPLES

Hereinafter, the present disclosure is described still more specifically by showing Examples and Comparative Examples; however, the present disclosure is not limited to these Examples. Abbreviations of constituent components described in Examples and Comparative Examples are as follows. In the following, all descriptions of "parts" and "%" are on a mass basis unless otherwise specified. The unit of the SP value $(cal/cm^3)^{1/2}$ is omitted.

Various compounds and materials described in Examples and Comparative Examples are as follows.

(1) Multifunctional (meth)acrylamide (A)

A-1: Polypropylene oxydimethacrylamide (D400) (n = an integer of 1 to 2, SP value = 10.6, average molecular weight = 366, acrylic equivalent = 183)
A-2: Polypropylene oxydiacrylamide (D400) (n = an integer of 2 to 3, SP value = 10.4, average molecular weight = 400, acrylic equivalent = 200)
A-3: Polypropylene oxydiacrylamide (D2000) (n = 33, SP value = 9.0, average molecular weight = 2100, acrylic equivalent = 1050)
A-4: Polypropylene oxydiacrylamide (D4000) (n = 67, SP value = 8.8, average molecular weight = 4100, acrylic equivalent = 2050)
A-5: Trimethylolpropane polypropylene oxytriacrylamide (D600) (x2 = an integer of 1 to 2, y2 = an integer of 1 to 2, z2 = an integer of 1 to 2, x2 + y2 + z2 = 5 to 6, m = 1, SP value = 10.5, average molecular weight = 600, acrylic equivalent = 200)
A-6: Trimethylolpropane polypropylene oxytriacrylamide (D3000) (x2 = an integer of 1 to 20, y2 = an integer of 1 to 10, z2 = an integer of 1 to 20, x2 + y2 + z2 = 50, m = 0, SP value = 9.0, average molecular weight = 3200, acrylic equivalent = 1067)
A-7: Trimethylolpropane polypropylene oxytriacrylamide (D5000) (x2 = an integer of 1 to 30, y2 = an integer of 1 to 25, z2 = an integer of 1 to 30, x2 + y2 + z2 = 84, m = 0, SP value = 8.9, average molecular weight = 5200, acrylic equivalent = 1733)
A-8: Polypropylene oxy polyethylene oxydiacrylamide (D700) (x1 = an integer of 1 to 2, y1 = an integer of 1 to 9, z1 = an integer of 1 to 2, x1 + z1 = an integer of 2 to 4, SP value = 10.0, average molecular weight = 700, acrylic equivalent = 350)
A-9: Polypropylene oxy polyethylene oxydiacrylamide (D1000) (x1 = an integer of 1 to 3, y1 = an integer of 1 to 13, z1 = an integer of 1 to 3, x1 + z1 = an integer of 2 to 6, SP value = 9.7, average molecular weight = 1000, acryl equivalent = 500)
A-10: Polypropylene oxy polyethylene oxydiacrylamide (D2000) (x1 = an integer of 1 to 3, y1 = an integer of 1 to 39, z1 = an integer of 1 to 3, x1 + z1 = an integer of 2 to 6, SP value = 9.5, average molecular weight = 2100, acrylic equivalent = 1050)
A-11: Pentaerythritol polypropylene oxytetraacrylamide (D800) (x3 = an integer of 1 to 3, y3 = an integer of 1 to 3, z3 = an integer of 1 to 3, s3 = an integer of 1 to 3, x3 + y3 + z3 + s3 = 8, SP value = 10.7, average molecular weight = 813, acrylic equivalent = 203)

(2) Polymerizable Compounds (B)

(2-1) Monofunctional Polymerizable Compounds (b1)

b1-1: Acryloylmorpholine (registered trademarks "Kohshylmer" and "ACMO", SP value = 11.2, water-soluble)
b1-2: N-hydroxyethylacrylamide (registered trademarks "Kohshylmer" and "HEAA", SP value = 14.4, water-soluble)
b1-3: Dimethylacrylamide (registered trademarks "Kohshylmer" and "DMAA" SP value = 10.6 water-soluble)
b1-4: Diethylacrylamide (registered trademarks "Kohshylmer" and "DEAA" SP value = 10.1 water-soluble)
b1-5: Isopropylacrylamide (registered trademarks "Kohshylmer" and "NIPAM" SP value = 10.6 water-soluble)
b1-6: Diacetone acrylamide (registered trademark "Kohshylmer", SP value = 10.7, water-soluble)
b1-7: N,N-dimethylamidopropylacrylamide (registered trademarks "Kohshylmer" and "DMAPAA", SP value = 10.3, water-soluble).
b1-8: N-hydroxyethylmethacrylamide (registered trademark "Kohshylmer", SP value = 13.8, water-soluble)
b1-9: N-methyl-N-hydroxyethylacrylamide (registered trademark "Kohshylmer", SP value = 13.3, water-soluble)

b1-10: N-octyl acrylamide (registered trademark "Kohshylmer", SP value = 9.9, water-insoluble)
b1-11: Isobornyl acrylate (SP value = 8.7, water-insoluble)
b1-12: 4-t-Butylcyclohexyl acrylate (registered trademark "Kohshylmer", SP value =8.7, water-insoluble)
b1-13: Butyl acrylate (SP value = 8.8, water-insoluble)
b1-14: 2-Ethylhexyl acrylate (SP value = 8.6, water-insoluble)
b1-15: Tetrahydrofurfuryl acrylate (SP value = 9.5, water-soluble)
b1-16: Hydroxyethyl acrylate (SP value = 12.5, water-soluble)
b1-17: 4-Hydroxybutyl acrylate (SP value = 11.7, water-soluble)
b1-18: Phenoxyethyl acrylate (SP value = 10.1, water-insoluble)
b1-19: Acrylic acid (SP value = 11.1, water-soluble)
b1-20: Isobornyl methacrylate (SP value = 8.7, water-insoluble)
b1-21: Acryloylaminoethyltrimethylammonium trifluoromethanesulfonate (SP value = 8.3, water-insoluble)
b1-22: Acryloylaminopropyltrimethylammonium bis (trifluoromethanesulfonyl) imide (SP value = 11.1, water-insoluble)

(2-1) Polyfunctional Polymerizable Compounds (b2)

b2-1: 1,6-Hexanediol diacrylate (SP value = 9.6, water-insoluble)
b2-2: Dipentaerythritol hexaacrylate (SP value = 10.4, water-insoluble)
b2-3: Polyethylene glycol 400 diacrylate (SP value = 9.6, water-soluble)
b2-4: Pentaerythritol triacrylate (SP value = 11.5, water-insoluble)
b2-5: Bifunctional urethane acrylate UV-3000B (estimated SP value = 10.0 (polyurethane SP value reference), water-insoluble).
b2-6: Bifunctional urethane acrylate UV-6640B (estimated SP value = 10.0 (polyurethane SP value reference), water-insoluble).
b2-7: Methylenebisacrylamide (SP value = 13.4, water-soluble)

(3) Polymerizable Polymerization Initiators (C)

C-1: 2-(4-(2-Hydroxy-2-methylpropionyl) phenoxy) ethyl acrylate (water-insoluble)
C-2: 2-Phenylphenacyl acrylate (water-insoluble)
C-3: 4-Acryloyloxy benzophenone (water-insoluble)
C-4: Kohshyex-I 3002 (Polymer type, water-insoluble) (registered trademark "Kohshyex", manufactured by KJ Chemicals Co., Ltd).

(4) Other Components (E)

(4-1) Additives (non-polymerizable polymerization initiator, sensitizer, pigment, etc.)

E1-1: Omnirad 2595 (water-soluble photopolymerization initiator)
E1-2: Omnirad TPO (water-insoluble photopolymerization initiator)
E1-3: Omnirad 1173 (water-insoluble photopolymerization initiator)
E1-4: Tuckyfire E-359 (non-polymerizable hydrogenated rosin, manufactured by Arakawa Chemical Industries, Ltd)
E1-5: Carbon black (pigments, C.I.Pigment Black 7)
E1-6: Solsperse 6000 (Pigment dispersant, manufactured by Lubrizol Japan Corporation)
E1-7: 2,4-Diethyl thioxanthone (sensitizer, manufactured by Lambson Co.)
E1-8: BYK-377 (silicone-based surface conditioner, solid content 100%, manufactured by BYK Japan)
E1-9: Anthox MS60 (sulfonate-based emulsifier, Nippon Nyukazai Co., Ltd)
E1-10: 2,2'-azobis(N-(2-carboxyethyl)-2-methyl propionamidine) tetrahydrate (water-soluble, thermal polymerization initiator)
E1-11: Pigments blue 15:4 (water-soluble pigments, manufactured by Toyo Color Co., Ltd)
E1-12: BYKJET-9151 (water-soluble pigment dispersant, manufactured by BYK)
E1-13: Inorganic filler (titanium dioxide)

(4-2) Organic Solvents, Quaternary Salt Monomer, etc.

E2-1: Ethyl acetate

E2-2: Methyl ethyl ketone
E2-3: Toluene
E2-4: Acryloylaminopropyltrimethylammonium p-toluenesulfonate
E2-5: Acryloyloxyethyltrimethylammonium bis (trifluoromethanesulfonyl) imide

(5) Water-soluble Polyfunctional (Meth)acrylamide and Aater-insoluble Polyfunctional (meth) acrylate

F-1: Polyethylene glycol diacrylamide (n = 2) (molecular weight = 256, acryl equivalent = 128, SP value = 11.8, water-soluble)
F-2: N-(tris (3-acrylamidopropoxymethyl) methyl) acrylamide (molecular weight = 508, acrylic equivalent = 127, SP value = 12.1, water soluble)
F-3: N,N'-(oxybis (2,1-ethanediyloxy-3,1-propanediyl)) bisacrylamide (molecular weight = 328, acryl equivalent = 164, SP value = 11.1, water soluble)
F-4: Tripropylene glycol diacrylate (n = 3) (molecular weight = 300, acrylic equivalent = 150, SP value = 9.3, water-insoluble)

(6) Abbreviation of Evaluation Substrate

PET: Easy adhesion polyethylenetelephthalate plate and film
PMMA: Polymethyl methacrylate plate and film
PC: Polycarbonate plate and film
PVC: Polyvinylchloride plate and film
ABS: Acrylonitrile-butadiene-styrene copolymer synthetic resin plate
GL: Glass plate
AL: Aluminum plate

Examples 1 to 15 and Comparative Examples 1 to 6 (Preparation and Evaluation of Curable Composition)

The polyfunctional (meth)acrylamide (A), the polymerizable compound (B), and other components were weighed in mass ratios shown in Table 1 and mixed at 35 °C for 1 hour to prepare curable compositions 1 to 15 and Comparative Examples 1 to 6. The transparency (compatibility) and the active energy ray curability of the obtained curable compositions were evaluated by the following methods, and the results are shown in Table 1. Table 1 also shows the SP values of (A) and (B) and the absolute value of the difference between the SP values of (A) and (B).

Evaluation of Transparency (Compatibility) of Curable Composition

The obtained various curable compositions were allowed to stand at 23 °C overnight, the states of the compositions were visually observed, and the transparency was evaluated in four grades as described below.

◎: The transparency was high, and no white turbidity or separation was observed.
○: The transparency is high, but white turbidity is slightly observed.
△: There was no phase separation, but there was turbidity.
×: There was white turbidity and phase separation

Evaluation of Active Energy Ray Curability of Curable Compositions

A PET film with a thickness of 100 μm (manufactured by Toyobo Co., Ltd., polyester film COSMOSHINE A4100) was brought into close contact with a horizontally placed glass plate with the easy-adhesion side of the PET film faced the front side, and the curable compositions of each Example and Comparative Example were applied using a bar coater (No. 12) so that the coating film thickness after drying would be 20 μm. After heating for 2 minutes at 60 °C, the curable composition was cured by irradiation with ultraviolet rays (apparatus: ITEC System Co., Ltd., tabletop batch type UV-LED curing device MUVBA-0.3 × 0.3 × 0.5, wavelength 405 nm, illumination (UV-V) 50 mW/$cm^2$). Thereafter, the presence or absence of tackiness on the surface of the cured film was confirmed, ultraviolet irradiation was additionally performed until the tackiness disappeared, and the curability was evaluated according to the following criteria based on the required integrated light amount.

◎: The tack disappeared when the integrated light amount was less than 500 mJ/$cm^2$.
○: The tack disappeared when the integrated light amount was 500 mJ/$cm^2$ or more and less than 1000 mJ/$cm^2$.

△: The tack disappeared when the integrated light amount was 1000 mJ/cm$^2$ or more and less than 2000 mJ/cm$^2$.
×: Tack remained even when the integrated light amount was 2000 mJ/cm$^2$ or more.

[Table 1]

| | | Curable Composition (mass%) | | | | | | | | | | SP Value of A | SP Value of B | Absolute Value of the Difference in the SP Value Between A and B | Transparency | Curability |
|---|---|---|---|---|---|---|---|---|---|---|---|---|---|---|---|---|
| | | A | | B | | | | C | | E | | | | | | |
| | | Type | Mass (%) | Type | Mass (%) | Type | Mass (%) | Type | Mass (%) | Type | Mass (%) | | | | | |
| Examples | 1 | a-1 | 92 | b1-1 | 7 | | | c-1 | 1 | | | 10.6 | 11.2 | 0.6 | ◎ | ◎ |
| | 2 | a-2 | 50 | b1-2<br>b1-3 | 24<br>8 | b2-7 | 10 | | | E1-1 | 8 | 10.4 | 13.4 | 3.0 | ◎ | ◎ |
| | 3 | a-3 | 80 | b1-3 | 14 | b2-1 | 3 | c-2 | 2.5 | E2-4 | 0.5 | 9.0 | 10.4 | 1.4 | ◎ | ◎ |
| | 4 | a-4 | 20 | b1-4<br>b1-12 | 40<br>15 | b2-2 | 20 | c-3 | 3 | E1-3 | 2 | 8.8 | 9.9 | 1.1 | ◎ | ◎ |
| | 5 | a-5 | 40 | b1-5<br>b1-16 | 5<br>35 | b2-3 | 15 | c-2 | 5 | | | 10.5 | 11.5 | 1.0 | ◎ | ◎ |
| | 6 | a-6 | 60 | b1-7<br>b1-17 | 10<br>10 | b2-2 | 10 | c-2 | 10 | | | 9.0 | 10.8 | 1.8 | ◎ | ◎ |
| | 7 | a-7 | 5 | b1-6<br>b1-15 | 10<br>42 | b2-4 | 40 | c-1 | 2 | E1-2 | 1 | 8.9 | 10.5 | 1.6 | ◎ | ◎ |
| | 8 | a-8 | 80 | b1-8<br>b1-20 | 2<br>15 | b2-7 | 1 | | | E1-3 | 2 | 10.0 | 9.5 | 0.5 | ◎ | ◎ |
| | 9 | a-9 | 60 | b1-9<br>b1-17 | 23<br>10 | | | c-1<br>c-2 | 2<br>4. | E1-8 | 0.1 | 9.7 | 12.8 | 3.1 | ○ | ◎ |
| | 10 | a-10 | 10 | b1-10<br>b1-11 | 5<br>75 | b2-3 | 5 | c-2 | 0.1 | E1-3 | 4.9 | 9.5 | 8.8 | 0.7 | ◎ | ○ |
| | 11 | a-11 | 1 | b1-1<br>b1-17 | 43<br>14 | b2-1<br>b2-3 | 10<br>30 | c-3 | 1.2 | E1-4 | 0.8 | 10.7 | 10.6 | 0.1 | ◎ | ◎ |
| | 12 | a-1 | 10 | | | b2-1<br>b2-6 | 30<br>10 | c-1 | 10 | E2-2 | 40 | 10.6 | 9.7 | 0.9 | ◎ | ◎ |
| | 13 | a-4<br>a-10 | 25<br>5 | b1-10<br>b1-18 | 25<br>30 | b2-4 | 10 | c-3 | 1 | E1-3 | 4 | 8.9 | 10.2 | 1.3 | ◎ | ◎ |
| | 14 | a-5 | 15 | b1-4<br>b1-14 | 40<br>10 | b2-1<br>b2-6 | 25<br>7 | c-1 | 3 | | | 10.5 | 9.8 | 0.7 | ◎ | ◎ |
| | 15 | a-3 | 8 | b1-3 | 50 | b2-2 | 34 | | | E1-1<br>E2-5 | 3<br>5 | 9.0 | 10.5 | 1.5 | ◎ | ◎ |
| Comparative Examples | 1 | f-1 | 20 | b1-4<br>b1-12 | 35<br>20 | b2-2 | 20 | c-3 | 3 | E1-3 | 2 | 11.8 | 9.8 | 2.0 | △ | △ |
| | 2 | a-10 | 95 | | | | | c-1 | 5 | | | 9.5 | - | - | × | ◎ |
| | 3 | f-2 | 5 | b1-4<br>b1-14 | 40<br>40 | b2-6 | 10 | c-1 | 3 | E1-3 | 2 | 12.1 | 9.4 | 2.7 | △ | △ |
| | 4 | | | b1-1<br>b1-18 | 40<br>20 | b2-3<br>b2-5 | 20<br>15 | c-1 | 2 | E1-3 | 3 | - | 10.3 | - | × | △ |
| | 5 | f-3 | 30 | b1-3 | 40 | b2-4 | 25 | | | E1-3 | 5 | 11.1 | 10.9 | 0.2 | × | △ |
| | 6 | f-4 | 10 | b1-6<br>b1-15 | 20<br>60 | b2-4 | 7 | c-1 | 2.5 | E1-2 | 0.5 | 9.3 | 9.9 | 0.6 | × | × |

As is clear from the results in Table 1, the curable composition (D) containing the water-insoluble polyfunctional (meth)acrylamide (A) and the polymerizable compound (B) had excellent transparency and curability. Although (A) is

water-insoluble, it can be compatibilized with (B), which is hydrophobic, hydrophilic and water-soluble, because it exhibits amphiphilic properties. The SP value of (A) was 8.8 to 11.0, the SP value of (B) was 8.5 to 14.5, and when the difference between the SP values of (A) and (B) was 3.0 or less, the transparency of (D) obtained was higher. The content of (A) is 1 to 95% by mass and the content of (B) is 5 to 99% by mass with respect to the total mass of (D), and it was found that both transparency and curability of (D) are favorable by containing a monofunctional polymerizable compound (b1) and/or a polyfunctional polymerizable compound (b2) as (B), and in particular, when the total content of (A) and (b2) exceeded 15% by mass, curability of (D) became higher. On the other hand, when only (A) (Comparative Example 2) or only (B) (Comparative Example 4) was contained, the compatibility of (A) or (B) with the polymerization initiator was insufficient, and thus the transparency of the curable composition was poor. Further, when (A) was not contained (Comparative Example 4), when a water-soluble polyfunctional (meth)acrylamide was contained (Comparative Examples 1, 3 and 5), and when a water-insoluble polyfunctional acrylate was contained (Comparative Example 6), both the transparency and the curability of the curable composition were not satisfactory.

Examples 16 to 21 and Comparative Examples 7 and 8 (Preparation and Evaluation of Coating Agent Composition)

The curable composition obtained in Table 1, the polyfunctional (meth)acrylamide (A), the polymerizable compound (B), and other components were weighed in mass ratios shown in Table 2, and mixed at 35 °C for 1 hour to prepare coating agent compositions of Examples 16 to 21 and Comparative Examples 7 and 8. The wettability of the resulting coating composition to various substrates and resistance to cure shrinkage during active energy ray curing were evaluated by the following methods. In addition, a coating film (also referred to as a cured film or coated film) was produced on a PET film using the coating composition by the following method, and the tackiness resistance, appearance, and surface hardness of the obtained coating film were evaluated by the following methods. Various evaluation results are shown in Table 2.

Wettability Evaluation of Coating Agent Composition

The coating agent compositions of each Example and Comparative Example were applied to various substrates shown in Table 2 with a bar coater (No. 12), and the cissing of the coating liquids was visually observed.

◎: The coating film was uniform without cissing.
○: There was very little cissing, while the coating film was substantially uniform.
△: There was some cissing, while the coating film was substantially uniform as a whole.
×: The coating film had a large amount of cissing and was uneven.

Cure Shrinkage Resistance (Curling Resistance) Evaluation of Coating Agent Composition

The coating agent compositions of each Example and Comparative Example were applied to a PET film with a thickness of 100μm using a bar coater (No. 12), and a coating film with a thickness of 10 μm was produced by irradiation with ultraviolet rays (apparatus: inverter-type conveyor apparatus ECS 4011GX manufactured by Eye Graphics Co., Ltd., metal halide lamp: M04 L41 manufactured by Eye Graphics Co., Ltd., UV illuminance: 700 $mW/cm^2$, integrated light amount: 1000 $mJ/cm^2$). When a solvent was used, the coating agent composition was dried at 80 °C for 3 minutes and then irradiated with UV rays. The obtained coating film was cut into 10 cm corners, and the average of the uplift of the four corners was measured. The cure shrinkage resistance (curling resistance) was evaluated. The larger the lifting, the larger the curl and the lower the cure shrinkage resistance.

◎: There was a lifting of 0.5 mm or less.
○: There was a lifting of 1.0 mm or less.
△: There was a lifting of 3.0 mm or less.
×: There was a large curling.

Evaluation of Tackiness Resistance of Coating Film

Using each of the coating agent compositions of Examples and Comparative Examples, a coating film was prepared in the same manner as in the evaluation of the cure shrinkage resistance of the coating film by using a bar coater (No. 6) so that the film thickness after drying was 5 μm. The surface of the obtained coating film was contacted with a silicone rubber stopper, and the stickiness (surface tackiness) was evaluated according to the following criteria. The higher the stickiness, the lower the tack resistance.

◎: There was no stickiness.

○: There was slight stickiness, but no trace remains on the surface.

△: It was sticky and left marks on the surface.

×: Stickiness was severe, and the rubber stopper sticked to the surface.

Appearance Evaluation of Coating Film

Using each of the coating agent compositions of Examples and Comparative Examples, a coating film with a thickness of 10 μm was prepared in the same manner as in the evaluation of cure shrinkage resistance. The surface smoothness and transparency of the obtained coating film were visually observed, and the appearance of the coating film was evaluated according to the following criteria. The higher the surface smoothness and the higher the transparency of the coating film, the better the appearance.

◎:The surface was smooth, and the coating film was transparent.

○: The surface was smooth, and the coating film was entirely transparent with a slight white turbidity.

△: The surface was uneven, or the coating film had some white turbidity.

×: The surface was uneven, and the coating film was wide turbid.

Surface Hardness Evaluation of Coating Film

Using each of the curable compositions of Examples and Comparative Examples, a coating film with a thickness of 10 μm was prepared in the same manner as in the evaluation of cure shrinkage resistance. The obtained coating film was scratched with pencils at an angle of 45 ° by about 10 mm according to JIS K 5600, and then surface hardness was evaluated as follows.

◎: Pencil hardness was 2H or more.

○: Pencil hardness was HB to H.

△: Pencil hardness was 3B to B.

×: Pencil hardness was 4B or less.

[Table 2]

| | | Coating Agent Composition | | | | | | | | Property of Coating Agent Composition | | | | Properties of Coating Film | | | |
|---|---|---|---|---|---|---|---|---|---|---|---|---|---|---|---|---|---|
| | | Curable Composition | | A | | B | | Others | | Wettability | | | | Cure Shrinkage Resistance | Tackiness Resistance | Appearance | Surface Hardness |
| | | Type | Mass (%) | Type | Mass (%) | Type | Mass (%) | Type | Mass (%) | PET | PVC | ABS | AL | | | | |
| Examples | 16 | Example 2 | 60 | | | b1-1 | 13 | c-1 | 2 | ◎ | ◎ | ◎ | ◎ | ◎ | ○ | ◎ | ○ |
| | | | | | | b1-11 | 25 | | | | | | | | | | |
| | 17 | Example 4 | 10 | | | b1-1 | 50 | E1-2 | 3 | ◎ | ○ | ◎ | ○ | ○ | ◎ | ◎ | ◎ |
| | | | | | | b2-1 | 37 | | | | | | | | | | |
| | 18 | Example 5 | 10 | a-3 | 15 | b1-3 | 20 | c-2 | 5 | ◎ | ◎ | ◎ | ○ | ◎ | ◎ | ◎ | ◎ |
| | | | | | | b1-18 | 25 | | | | | | | | | | |
| | | | | | | b2-1 | 25 | | | | | | | | | | |
| | 19 | Example 6 | 100 | | | | | | | ◎ | ○ | ◎ | ◎ | ◎ | ◎ | ◎ | ◎ |
| | 20 | Example 15 | 20 | a-7 | 10 | b1-1 | 20 | c-3 | 10 | ◎ | ◎ | ○ | ○ | ◎ | ◎ | ◎ | ◎ |
| | | | | | | b1-20 | 25 | | | | | | | | | | |
| | | | | | | b2-1 | 15 | | | | | | | | | | |
| | 21 | Example 8 | 60 | | | b1-2 | 30 | c-4 | 10 | ◎ | ◎ | ◎ | ○ | ◎ | ◎ | ◎ | ○ |
| Comparative Examples | 7 | Comparative Example 1 | 100 | | | | | | | △ | × | △ | △ | × | △ | × | △ |
| | 8 | Comparative Example 5 | 10 | f-4 | 20 | b1-4 | 30 | E1-3 | 5 | △ | × | △ | △ | △ | △ | × | △ |
| | | | | | | b1-20 | 25 | | | | | | | | | | |
| | | | | | | b2-1 | 10 | | | | | | | | | | |

As is clear from the results in Table 2, the coating agent compositions of Examples contained the polyfunctional (meth)acrylamide (A) and the polymerizable compound (B), and exhibited good wettability to various substrates from plastic substrates (PET, PVC, and ABS) to metal (aluminum) while having high curability and cure shrinkage resistance. The coating films obtained from the coating agent compositions of Examples had good appearances, and the coating films had high tackiness resistance and surface hardness. These effects are due to the interaction of (A) and (B). The coating agent compositions of Comparative Examples which did not contain (A) and (B) at the same time were not confirmed to have similar properties.

Examples 22 to 27 and Comparative Examples 9 and 10 (Preparation and Evaluation of Pressure-sensitive Adhesive Composition)

The curable composition obtained in Table 1, the polyfunctional (meth)acrylamide (A), the polymerizable compound (B), the photopolymerization initiator (C), and other components were weighed in mass ratios shown in Table 3 and mixed at 35 °C for 1 hour to prepare the pressure-sensitive adhesive compositions of Examples 22 to 27 and Comparative Examples 9 and 10. Using the obtained pressure-sensitive adhesive composition, a pressure-sensitive adhesive layer and a pressure-sensitive adhesive sheet were produced by the following methods, and the curability of the pressure-sensitive adhesive composition, the adhesiveness to various substrates, and the pressure-sensitive adhesive force, transparency, stain resistance (reworkability), yellowing resistance, and moist-heat resistance of the obtained pressure-sensitive adhesive layer were evaluated, and the results are shown in Table 3.

Curability Evaluation of Pressure-sensitive Adhesive Composition

A heavy release PET film with a thickness of 75 μm (manufactured by Toyobo Co., Ltd., E7001 Polyester Films) is brought into close contact with a horizontally placed glass plate, and a spacer having a thickness of 1 mm and an interior of 60 mm × 100 mm was installed, each of the prepared curable pressure-sensitive adhesive compositions of Examples and Comparative Examples is filled inside the spacer, and a light release PET film (manufactured by Toyobo Co., Ltd., polyester film E7002) with a thickness of 50 μm was further stacked thereon, and irradiation is performed by a UVLED lamp having a wave length 385 nm and a power of 100 mW/cm$^2$ so that the integrated light amount is 3000 mJ/cm$^2$, and the pressure-sensitive adhesive composition was cured. Thereafter, the release PET films on both sides were removed, and the obtained cured product (pressure-sensitive adhesive layer) was touched with a finger to evaluate the curability in three grades according to the following criteria.

○: A cured product that maintains its shape was obtained, and tackiness was observed when the cured product was touched, but there was no adhesion of the liquid uncured product.
△: A cured product that maintains its shape was obtained, and tackiness was observed when the cured product was touched, but there was adhesion of a liquid uncured product.
×: This is a state in which curing was insufficient, a cured product which maintains a shape could not be obtained, and adhesion of a liquid residue was observed in a large amount.

Preparation of pressure-sensitive adhesive Sheet and Adhesion Evaluation

The pressure-sensitive adhesive compositions of Examples and Comparative Examples were applied onto various plate-shaped substrates (film or plate), laminated with light release separators (silicone-coated PET films) using a desktop roll laminator (RSL 382S, manufactured by Royal Sovereign) so that the pressure-sensitive adhesive layer had a thickness of 5 μm, and irradiated with UV rays (apparatus: inverter-type conveyor apparatus ECS 4011GX, manufactured by Eye Graphics Co., Ltd., metal halide lamp: M04 L41, manufactured by Eye Graphics Co., Ltd., UV irradiance: 700 mW/cm$^2$, integrated light quantity: 5000 mJ/cm$^2$). Thereafter, the light release separator was peeled off to obtain a pressure-sensitive adhesive sheet composed of the pressure-sensitive adhesive layer and the substrate. Using the obtained pressure-sensitive adhesive sheet, 100 squares with a 1 mm$^2$ were cross-cut with a cutter knife in accordance with JIS K 5600, a cellophane tape was attached, and the number of squares in which the pressure-sensitive adhesive layer remained on the substrate side when the tape was peeled off at once was counted, and the adhesion was evaluated according to the following criteria. The larger the number of remaining squares, the higher the adhesion.

◎:The number of remaining squares was 100.
○: The number of remaining squares was 95 to 99.
△: The number of remaining squares was 70-9 squares.
×: The number of remaining squares was 0 to 6.

Adhesive Strength Evaluation

Under the conditions of a temperature of 23 °C and a relative humidity of 50%, the pressure-sensitive adhesive layer was transferred to various film-like or plate-like substrates, pressure-bonded thereto by reciprocating twice using a pressure-bonding roller having a weight of 2 kg, and allowed to stand under the same atmosphere for 30 minutes. Thereafter, the 180° peel strength (N/25 mm) was measured at a peel rate of 300 mm/min in accordance with JIS Z0237 using a tensile tester (device name: Tensilon RTA-100 manufactured by ORIENTEC Co., Ltd). The higher the peel strength, the higher the adhesive strenght.

◎: 30 (N/25 mm) or more
○: 15 (N/25 mm) or more and less than 30 (N/25 mm)
△: 8 (N/25 mm) or more and less than 15 (N/25 mm)
×: less than 8 (N/25 mm)

Transparency Evaluation of Adhesive Layer

The total light transmittance of the glass substrate was measured in accordance with JIS K 7105 using a haze meter (NDH 2000, manufactured by Nippon Denshoku Industries Co., Ltd). The pressure-sensitive adhesive layer was transferred to a glass substrate under conditions of a temperature of 23 °C and a relative humidity of 50%, and the total light transmittance of the glass substrate and the adhesive layer was measured. Thereafter, the transmittance of the pressure-sensitive adhesive layer itself was calculated by subtracting the transmittance of the glass plate, and the transparency was evaluated in four grades as described below. The higher the transmittance, the higher the transparency.

◎: The transmissivity was 90% or more.
○: The transmissivity was 85% or more and less than 90%.
△: The transmissivity was 50% or more and less than 85%.
×: The transmissivity was less than 50%.

Stain Resistance (Reworkability) Evaluation of Adhesive Layer

A pressure-sensitive adhesive sheet was prepared in the same manner as in the measurement of the pressure-sensitive adhesive strength described above and allowed to stand at 80 °C for 24 hours. Thereafter, contamination (remaining state of the adhesive layer (glue)) on the surface of the substrate film after peeling off the adhesive layer was visually observed. The less the adhesive residue, the higher the stain resistance.

◎: No stain (no glue residue).
○: There was very little contamination.
△: There was slight contamination.
×: There was contamination (there was glue residue).

Yellowing Resistance Evaluation of Adhesive Layer

A pressure-sensitive adhesive sheet was prepared in the same manner as in the measurement of the pressure-sensitive adhesive strength described above, set in a xenon fade meter (SC-700-WA: manufactured by Suga Test Instruments). After irradiation with UV rays with an illuminance as 70 mW/$cm^2$ for 120 hours, discoloration of the pressure-sensitive adhesive layer on the pressure-sensitive adhesive sheet was visually observed.

◎: Yellowing could not be visually observed at all.
○: Yellowing was only slightly visible.
△: Yellowing could be visually confirmed.
×: A distinct yellowing could be detected visually.

Moist-heat Resistance Evaluation of Adhesive Layer

A pressure-sensitive adhesive sheet was produced in the same manner as in the measurement of the pressure-sensitive adhesive strength described above and was held under the conditions of a temperature of 85 °C and a relative humidity of 85% for 100 hours, and then the presence or absence of lifting or peeling of the pressure-sensitive adhesive layer, bubbles, or white turbidity was visually observed and evaluated.

◎: It was transparent and did not cause floating, peeling, or air bubbles.
○: There was very little haze, but no floating, peeling, or air bubbles bubbling.
△: There was slight clouding or lifting, flaking, and air bubbles.
×: There was extreme clouding or lifting, flaking, and air bubbles.

[Table 3]

| | | Pressure-sensitive Adhesive Composition | | | | | | | | Properties of Pressure-sensitive Adhesive Coating Film | | | | | | | | | | | |
|---|---|---|---|---|---|---|---|---|---|---|---|---|---|---|---|---|---|---|---|---|---|
| | | Curable Composition | | A | | B | | Others | | Curability | Adhesion | | | | Adhesive Strength | | | Transparency | Stain Resistance | Yellowing Resistance | Moist-heat Resistance |
| | | Type | Mass (%) | Type | Mass (%) | Type | Mass (%) | Type | Mass (%) | | PET | PMMA | ABS | GLASS | PET | PC | ABS | | | | |
| Examples | 22 | Example 1 | 40 | | | b1-14 | 58 | E1-2 | 2 | ○ | ◎ | ◎ | ◎ | ◎ | ◎ | ◎ | ◎ | ◎ | ◎ | ◎ | ◎ |
| | 23 | Example 3 | 22 | | | b1-13<br>b1-14 | 30<br>40 | c-2 | 8 | ○ | ◎ | ◎ | ◎ | ◎ | ◎ | ◎ | ◎ | ◎ | ◎ | ◎ | ◎ |
| | 24 | Example 6 | 10 | | | b1-13<br>b1-14 | 45<br>35 | c-4 | 10 | △ | ◎ | ◎ | ○ | ○ | ◎ | ◎ | ○ | ◎ | ○ | ◎ | ○ |
| | 25 | Example 9 | 20 | a-2 | 10 | b1-13<br>b1-15 | 30<br>38 | E1-3 | 2 | ○ | ◎ | ◎ | ◎ | ◎ | ◎ | ◎ | ◎ | ◎ | ◎ | ◎ | ◎ |
| | 26 | Example 13 | 50 | | | b1-13 | 40 | c-4 | 10 | ○ | ◎ | ◎ | ◎ | ○ | ◎ | ◎ | ◎ | ◎ | ○ | ◎ | ◎ |
| | 27 | Example 14 | 30 | | | b1-13<br>b1-14 | 40<br>27 | E1-1 | 3 | ○ | ◎ | ◎ | ◎ | ◎ | ◎ | ◎ | ◎ | ◎ | ○ | ◎ | ○ |
| Comparative Examples | 9 | Comparative Example 5 | 40 | f-2 | 12 | b1-13<br>b1-14 | 30<br>16 | E1-2 | 2 | △ | △ | × | × | × | × | × | × | × | △ | × | △ |
| | 10 | Comparative Example 6 | 70 | | | b1-13 | 25 | c-3 | 5 | × | △ | × | × | × | × | × | × | △ | × | △ | × |

As is clear from the results in Table 3, the pressure-sensitive adhesive compositions of Examples contained the polyfunctional (meth)acrylamide (A) and the polymerizable compound (B), and due to the interaction between them, the pressure-sensitive adhesive compositions exhibited high curability, and the pressure-sensitive adhesive layers obtained by curing the compositions had high transparency. In addition, the pressure-sensitive adhesive compositions of Examples had good adhesion and adhesiveness (adhesive strength) to various materials from general-purpose plastics to inorganic glasses. The cured product (pressure-sensitive adhesive layer) obtained from the pressure-sensitive adhesive composition of Example maintained high transparency. The cured products had good stain resistance when they were peeled from the substrates. The yellowing resistance and the moist-heat resistance of the cured products were also good. On the other hand, in the compositions of Comparative Examples were found to have low curability, low adhesion and low adhesiveness to various materials. The transparency, the stain resistance, the yellowing resistance, and the moist-heat resistance of the cured products obtained by Comparative Examples were also low.

Examples 28 to 33 and Comparative Examples 11 and 12 (Preparation and Evaluation of Adhesive Composition)

The curable composition obtained in Table 1, the polyfunctional (meth)acrylamide (A), the polymerizable compound (B), the photopolymerization initiator (C), and other components were weighed in mass ratios shown in Table 4 and mixed at 35 °C for 1 hour to prepare the adhesive compositions of Examples 28 to 33 and Comparative Examples 11 and 12. The transparency of the obtained adhesive composition was evaluated by the following method. Further, the same kind or different kinds of plate-like substrates are bonded by the following method by using the adhesive compositions of the respective Examples and Comparative Examples to prepare adhesive test pieces, and the adhesive strength, water resistance and impact resistance are evaluated, the results are shown in Table 4.

Transparency Evaluation of Adhesive Composition

The obtained adhesive composition was allowed to stand at 23 °C overnight, and then visually observed, and the transparency was evaluated in four grades as described below.

◎: The transparency was high, and no white turbidity or separation was observed.
○: The transparency was high, but white turbidity was slightly observed.
△: The layers were not separated, but were cloudy.
×: White turbidity and phase separation were observed.

Preparation of Adhesive Evaluation Test Pieces (Adhesive Test Pieces)

The adhesive composition was uniformly applied to any one of two plate-shaped substrates of the same type or different types having a length of 100 mm, a width of 25 mm, and a thickness of 1 mm. When a solvent was contained in the adhesive composition, the mixture was applied in a larger amount so that the thickness after drying was about the same as in the case of no solvent, and dried at 90 °C for 2 minutes. Thereafter, according to JIS K 6850, the other one of the plate-shaped substrates was placed on the coated adhesive composition, and the plate-shaped substrates were bonded to each other so that the overlapping region was 12. 5 mm in length and 25 mm in width, and the thickness of the adhesive layer was adjusted to 100 $\mu$m by using a spacer, thereby preparing a bonded test piece. Thereafter, UV rays (ultraviolet ray irradiation apparatus: inverter-type conveyor apparatus ECS 4011GX manufactured by Eye Graphics Co., Ltd., metal halide lamp: M04 L41 manufactured by Eye Graphics Co., Ltd., UV ray illumination: 700 mW/cm$^2$, integrated light amount: 5000 mJ/cm$^2$) were irradiated from the upper surface of the bonded transparent or translucent substrates. The irradiated test piece was used as an adhesive test piece.

Adhesive Strength Evaluation

The tensile shear strength of the obtained adhesive test piece was measured according to JIS K 6850 at a tensile speed of 10 mm/min using Tensilon RTA-100 (manufactured by ORIENTEC Co., Ltd) as a tester. The higher the tensile shear strength, the higher the adhesive force.

◎: The tensile shear strength was 20 Mpa or more.
○: The tensile shear strength was 15 MPa or more and less than 20 MPa.
△: The tensile shear strength was 10 MPa or more and less than 15 MPa.
×: The tensile shear strength was less than 10 MPa.

Water Resistance of Adhesive Test Piece

The obtained adhesive test piece was immersed in warm water at 60 °C for 48 hours, and then the presence or absence of interface peeling of the same or different plate-shaped substrate of was confirmed and evaluated according to the following criteria.

◎: There was no interfacial peeling (less than 1 mm).
○: There was slight interfacial peeling (1mm or more and less than 3 mm).
△: There was a little interfacial peeling (3mm or more, less than 5mm)
×: There was interfacial peeling (5mm or more).

Impact Resistance Evaluation of Adhesive Test Piece

Using the obtained adhesive test piece, the impact peel adhesive strength was measured using an impact tester No. 511 (manufactured by Sumitomo Testing Machine Co., Ltd) in accordance with JIS K6855. The higher the impact peel adhesive strength, the higher the impact resistance.

◎: The impact peel adhesive strength was 20 KJ/m$^2$ or more.
○: The impact peel adhesive strength was 15 KJ/m$^2$ or more and less than 20 KJ/m$^2$.
△: The impact peel adhesive strength was 10 KJ/m$^2$ or more and less than 15 KJ/m$^2$.
×: The impact peel adhesive strength was less than 10 KJ/m$^2$.

[Table 4]

| | | Adhesive Composition | | | | | | | | Properties of Adhesive Composition | Properties of Adhesive Layers | | | |
|---|---|---|---|---|---|---|---|---|---|---|---|---|---|---|
| | | Curable Composition | | A | | B | | Others | | | Adhesive Strength | | Water Resistance | Impact Resistance |
| | | Type | Mass (%) | Type | Mass (%) | Type | Mass (%) | Type | Mass (%) | Transparency | Substrates | Adhesive Strength | | |
| Examples | 28 | Example 4 | 20 | | | b1-1<br>b1-13<br>b2-1 | 30<br>20<br>20 | c-1 | 10 | ◎ | PET/PMMA | ○ | ○ | ○ |
| | 29 | Example 6 | 80 | | | b1-3<br>b2-2 | 10<br>10 | | | ◎ | PET/PET | ◎ | ◎ | ◎ |
| | 30 | Example 8 | 80 | | | b1-14<br>b2-4 | 10<br>8 | E1-3 | 2 | ○ | PET/PVC | ◎ | ◎ | ◎ |
| | 31 | Example 9 | 40 | a-2 | 6 | b1-1<br>b2-1 | 24<br>20 | c-4 | 10 | ◎ | PET/PC | ◎ | ◎ | ◎ |
| | 32 | Example 13 | 50 | | | b1-2<br>b1-14<br>b2-4 | 20<br>20<br>8 | E1-2 | 2 | ◎ | PET/Al | ◎ | ○ | ○ |
| | 33 | Example 15 | 60 | a-5 | 30 | b1-1 | 8 | E1-1 | 2 | ◎ | ABS/ABS | ◎ | ◎ | ◎ |
| Comparative Examples | 11 | Comparative Example 1 | 80 | | | b1-14<br>b2-4 | 10<br>8 | E1-3 | 2 | △ | PET/PVC | △ | × | △ |
| | 12 | Comparative Example 5 | 100 | | | | | | | × | PET/PET | × | △ | △ |

As is clear from the results in Table 4, the adhesive compositions of Examples contained the polyfunctional (meth) acrylamide (A) and the polymerizable compound (B), and exhibited high transparency. Further, the adhesive compositions of Examples were used for bonding various same or different materials from plastic substrates (PET, PMMA, PC, PVC, ABS) to metal (aluminum), and sufficiently satisfactory adhesive strength was obtained. This is because the adhesive composition was excellent in wettability to various materials, and at the same time, the adhesive strength of the adhesive obtained after curing was high. In addition, by having (A) containing many isopropyleneoxy groups, the water resistance and the impact resistance of the adhesive obtained in Examples were good. Examples 29 to 31 contained (A) in which isopropyleneoxy group having a branched structure, further improvement of the water resistance and the impact resistance was confirmed. On the other hand, the adhesive compositions of Comparative Examples which did not contain (A) and (B) at the same time and the adhesives obtained therefrom were not confirmed to have similar properties.

Examples 34 to 39 and Comparative Examples 13 and 14 (Preparation and Evaluation of Curable Ink Composition)

The curable composition obtained in Table 1, the polyfunctional (meth)acrylamide (A), the polymerizable compound (B), the photopolymerization initiator (C), and other components were weighed in mass ratios shown in Table 5, and mixed in 35°C for 1 hour to prepare the ink compositions of Examples 34 to 39 and Comparative Examples 13 and 14. Using the obtained ink composition, the viscosity was measured by the following method, and the transparency in the case of containing no pigment (clear ink) and the pigment dispersibility in the case of containing a pigment were evaluated. The curability of the ink composition and the ejection stability at the time of inkjet printing were evaluated, and the clarity of the obtained printed matter was evaluated. The results of these evaluations are summarized in Table 5.

Viscosity Measurement and Evaluation of Ink Composition

The viscosity of the obtained ink composition at 25 °C was measured using a cone-plate viscosimeter (RE550 viscosimeter manufactured by Toki Sangyo Co., Ltd) in accordance with JIS K5600-2-3. As an ink composition for ink jet printing, the viscosity was evaluated in four grades as follows.

◎: 5 or more and less than 100 mPa·s
○: 100 or more and less than 500 mPa·s
△: 500 or more and less than 2000 mPa·s
×: 2000 mPa·s S or more

Pigment Dispersibility Evaluation

Using the obtained ink composition, the state of aggregation or precipitation of the pigment was visually observed immediately after preparation and after standing for 2 months at room temperature, and the pigment dispersibility was evaluated according to the following criteria.

◎: Neither aggregation nor precipitation of the pigment was observed immediately after the preparation nor after standing for 2 months.
○: No precipitation was observed immediately after the preparation, but slight precipitation of the pigment was observed after standing for 2 months.
△: Slight aggregation or precipitation of the pigment was observed immediately after preparation, and clear aggregation or precipitation was observed after standing for 2 months.
×: Aggregation and precipitation of the pigment were clearly observed even immediately after the preparation.

Transparency Evaluation (Clear ink)

The obtained ink composition was put into a transparent screw tube, the initial state was visually observed, and the ink composition was allowed to stand in a dark place at room temperature for 24 hours. Thereafter, the state of the ink composition after standing was visually observed, and the compatibility was evaluated according to the following criteria.

◎: Neither phase separation nor white turbidity of the liquid was observed at the initial stage nor after standing.
○: Phase separation and white turbidity of the liquid were not observed at the initial stage, but phase separation or white turbidity was slightly observed after standing.
△: At the initial stage, slight layer separation or white turbidity was observed, and after standing, clear layer separation or white turbidity was observed.
×: Even at the initial stage, clear layer separation or white turbidity was observed.

Preparation of Printed matter by Irradiation with Active Energy Rays

The ink composition was applied to a PET film with a thickness of 100 μm using a bar coater (No. 12) (thickness after drying: 10 μm), and cured by UV irradiation (apparatus: inverter-type conveyor apparatus ECS 4011GX manufactured by Eye Graphics Co., Ltd., metal halide lamp: M04 L41 manufactured by Eye Graphics Co., Ltd., UV illuminance: 700 mW/cm$^2$, integrated light amount: 1000 mJ/cm$^2$) to produce a printed material.

Curability Evaluation of Ink Composition

A printed material was prepared by the above-described method, and the integrated light amount until the ink composition was completely cured (in a non-sticky state) was measured to evaluate the curability. The lower the required integrated amount of light, the higher the curability.

◎: completely cured at less than 1000mJ/cm$^2$
○: completely cured at 1000 or more and less than 2000 mJ/cm$^2$
△: completely cured at 2000 or more and less than 5000 mJ/cm$^2$
×: 5000 mJ/cm$^2$ or more was required until completely cured.

Inkjet Printing and Ink Ejection Stability Evaluation

The ink composition was filled in a commercially available ink jet printer (Luxel Jet UV350GTW, manufactured by FUJIFILM Corporation), a solid image was printed using coated paper, the printing state of the obtained printed matter was visually observed, and the ejection stability was evaluated according to the following criteria.

◎: Good printing was performed without nozzle omission.
○: Nozzle omission was slightly observed.
△: There was nozzle omission in a wide range.
×: There was non-ejection.

Print clarity Evaluation

The image clarity of the inkjet printed matter obtained from the ink composition containing the pigment was visually observed.

◎: No ink bleeding was observed, and the image was clear.
○: There was almost no ink bleeding, and the image was good.
△: Some ink bleeding was observed.
×: Significant ink bleeding was observed.

[Table 5]

| | | Ink Composition | | | | | | | | Properties of Ink Composition | | | Properties of Cured Ink | | |
|---|---|---|---|---|---|---|---|---|---|---|---|---|---|---|---|
| | | Curable Composition | | A | | B | | Others | | Viscosity | PigmentDdispersibility | Clear Ink Transparency | Curability | Ink Ejection Stability | Print clarity |
| | | Type | Mass (%) | Type | Mass (%) | Type | Mass (%) | Type | Mass (%) | | | | | | |
| Examples | 34 | Example 3 | 60 | | | b1-3 | 20 | E1-3 | 3 | ○ | – | ◎ | ◎ | ○ | ◎ |
| | | | | | | b2-2 | 17 | | | | | | | | |
| | 35 | Example 8 | 30 | a-2 | 5 | b1-1 | 26.5 | E1-2 | 3 | ◎ | ◎ | – | ◎ | ◎ | ◎ |
| | | | | | | b1-15 | 15 | E2-4 | 7 | | | | | | |
| | | | | | | b2-4 | 10 | E1-5 | 3 | | | | | | |
| | | | | | | | | E1-6 | 0.5 | | | | | | |
| | 36 | Example 10 | 70 | | | b1-1 | 10 | c-1 | 10 | ◎ | – | ◎ | ○ | ◎ | ○ |
| | | | | | | b2-2 | 10 | | | | | | | | |
| | 37 | Example 11 | 50 | a-5 | 30 | b1-4 | 14.5 | c-4 | 3 | ◎ | ◎ | – | ◎ | ◎ | ◎ |
| | | | | | | | | E1-5 | 2 | | | | | | |
| | | | | | | | | E1-6 | 0.5 | | | | | | |
| | 38 | Example 13 | 60 | a-9 | 10 | b1-1 | 22 | E1-5 | 2.5 | ◎ | ◎ | – | ◎ | ◎ | ◎ |
| | | | | | | b2-4 | 5 | E1-6 | 0.5 | | | | | | |
| | 39 | Example 6 | 70 | | | b1-2 | 20 | c-4 | 10 | ◎ | – | ◎ | ◎ | ◎ | ◎ |
| Comparative Examples | 13 | Comparative Example 3 | 100 | | | | | | | ◎ | – | △ | × | ○ | △ |
| | 14 | Comparative Example 5 | 50 | | | b1-2 | 25 | E1-2 | 2 | ◎ | × | – | ○ | × | × |
| | | | | | | b1-15 | 20 | E1-5 | 2.5 | | | | | | |
| | | | | | | | | E1-6 | 0.5 | | | | | | |

As is clear from the results in Table 5, the ink compositions of Examples contained the polyfunctional (meth) acrylamide (A) and the polymerizable compound (B) with high compatibility, had high transparency (clear ink) or high dispersibility of pigments. In addition, the curability of the ink composition containing (A) and (B) was high, the viscosity of the ink composition for ink jet printing could be prepared to be low, and the ejection stability and the print clarity of the obtained printed matter were also good. On the other hand, all the ink compositions of Comparative Examples had low viscosity, transparency, pigment dispersibility, curability, ejection stability, or the print clarity of the obtained printed matter was not satisfactory.

Examples 40 to 45 and Comparative Examples 15 and 16 (Preparation and Evaluation of Aqueous Ink Composition)

The curable composition obtained in Table 1, the polyfunctional (meth)acrylamide (A), the polymerizable compound (B), the photopolymerization initiator (C), and other components were weighed in mass ratios shown in Table 6, and mixed at 35 °C for 1 hour to prepare the aqueous ink compositions of Examples 40 to 45 and Comparative Examples 15 and 16. Using the obtained aqueous ink composition, the compatibility with water (clear ink) and the dispersibility of the water-soluble pigment were evaluated by the following methods. Further, the initial viscosity was measured to evaluate the

viscosity suitability as an ink composition for ink jet printing. The obtained aqueous ink composition was stored in a dark place at 40 °C for 1 month, the viscosity after storage was measured in the same manner, the change rate of the viscosity after storage with respect to the initial value was calculated by the following equation, and the storage stability of the ink was evaluated according to the following criteria. The curability of the aqueous ink composition and the ejection stability at the time of ink jet printing were evaluated, and the surface drying properties and clarity of the obtained printed matter were evaluated. The results of these evaluations are summarized in Table 6.

Viscosity change rate (%) = (viscosity after storage-initial viscosity)/initial viscosity × 100%

Compatibility Evaluation (Clear Ink)

The obtained aqueous ink composition was put into a transparent screw tube, the initial state was visually observed, and the aqueous ink composition was allowed to stand in a dark place at room temperature for 24 hours. Thereafter, the state of the aqueous ink composition after standing was visually observed, and the compatibility was evaluated according to the following criteria.

◎: Neither phase separation nor white turbidity of the liquid was observed at the initial stage or after standing.
○: Phase separation and white turbidity of the liquid were not observed at the initial stage, but phase separation or white turbidity was slightly observed after standing.
△: Slight layer separation or white turbidity was observed at the initial stage, and clear layer separation or white turbidity was observed after standing.
×: Clear layer separation or white turbidity was observed even at the initial stage.

Pigment Dispersibility Evaluation of Aqueous (Water-based) Ink Composition

The obtained aqueous ink composition containing aqueous pigment (pigment-containing aqueous ink composition) was placed in a transparent screw tube, the initial state was visually observed, and the composition was allowed to stand in a dark place at room temperature for 24 hours. Thereafter, the state of the aqueous pigment-containing aqueous ink composition after standing was visually observed, and the pigment dispersibility was evaluated according to the following criteria.

◎: No precipitation of the pigment was observed either at the initial stage or after standing.
O: No precipitation of the pigment was observed at the initial stage, but slight precipitation of the pigment was observed after standing.
△: Slight precipitation of the pigment was observed at the initial stage, and clear precipitation of the pigment was observed after standing.
×: At the initial stage, clear precipitation of the pigment was observed.

Viscosity Measurement and Evaluation of Aqueous Ink Composition

The viscosity of the obtained aqueous ink composition was measured using a cone-plate viscosimeter (RE550 viscosimeter manufactured by Toki Sangyo Co., Ltd) in accordance with JIS K5600-2-3. The viscosity suitability of the ink composition for ink jet printing was evaluated according to the following criteria.

◎: The viscosity was 5 mPa·s or more and less than 100 mPa·s.
O: The viscosity was 100 mPa·s or more and less than 500 mPa·s.
△: The viscosity was 500 mPa·s or more and less than 2000 mPa·s.
×: The viscosity was 2000 mPa·s or more.

Storage Stability Evaluation of Aqueous Ink Composition

The viscosity of the aqueous ink composition after storage in a dark place at 40 °C for one month was measured in the same manner, and the change rate of the viscosity was calculated and evaluated according to the following criteria.

◎: Viscosity change rate was ± less than 10%.
O: Viscosity change rate was ± 10% or more and less than ± 20%.
△: Viscosity change rate was ± 20% or more and less than ± 30%.
×: Viscosity change rate e ± 30% or more.

Preparation of Printed matter and Evaluation of Aqueous Ink Composition

Using the obtained aqueous ink composition, printing was performed on OK top coated paper (manufactured by Oji Paper Co., Ltd) with an ink jet evaluation apparatus (manufactured by K-SOLUTION Co., Ltd), and the printed matter was cured by irradiation with UV rays at an integrated light quantity (200mJ/$cm^2$) to obtain a printed matter. The ejection stability of the aqueous ink composition, and the surface dryness and water resistance of the obtained printed matter were evaluated according to the following criteria.

Evaluation of Ejection Stability of Aqueous Ink Composition

The printed state of the obtained printed matter was visually evaluated.

◎: Good printing was achieved with no missing nozzles.
O: Nozzle omission was slightly observed.
△: Nozzle omission was observed in a wide range.
×: Non-ejection occurred.

Curability Evaluation of Aqueous Ink Composition

The obtained printed matter was rubbed with a cotton swab, and the curability was evaluated according to the following criteria.

◎: No trace of rubbing with a cotton swab was left.
O: A slight scratch was left.
△: Rubbing marks remained in a wide range.
×: The printed matter was removed with a cotton swab.

Evaluation of Surface Dryness of Printed Matter

The obtained printed matter was allowed to stand in an environment of a room temperature of 23 °C and a relative humidity of 50% for 5 minutes, high quality paper was placed on the printed surface, a load of 1 kg/$cm^2$ was applied for 1 minute, and the degree of transfer of the ink to the paper was evaluated.

◎: The ink dried and there was no transfer to the paper.
O: The ink dried and there was slight transfer to the paper.
△: The ink was almost dry and there was slight transfer to the paper.
×: The ink was hardly dried, and much ink was transferred to the paper.

Water Resistance Evaluation of Aqueous Ink Composition

The obtained printed matter was rubbed with a cotton swab wetted with water, and the water resistance was evaluated according to the following criteria.

◎: Even after rubbing back and forth 40 times, no ink was left on the cotton swab and no scratches were observed on the printed surface.
O: After rubbing back and forth 40 times, there was a small amount of ink adhering to the cotton swab and scratches on the printed surface.
△: After rubbing back and forth 11 to 39 times, there was ink adhering to the cotton swab and scratches on the printed surface.
×: Even after rubbing back and forth 10 or less times, there was ink adhering to the cotton swab and scratches on the printed surface.

[Table 6]

| | | Aqueous Ink Composition | | | | | | | | | | Properties of Aqueous Ink Composition | | | | | Properties of Printed | |
|---|---|---|---|---|---|---|---|---|---|---|---|---|---|---|---|---|---|---|
| | | Curable Composition | | A | | B | | Water | Others | | | | | | | | | |
| | | Type | Mass (%) | Type | Mass (%) | Type | Mass (%) | Mass (%) | Type | Mass (%) | Compatibility (clear ink) | pigment dispersibility | Viscosity | Storage Stability | Ink Ejection Stability | Surface Dryness | Water Resistance |
| Examples | 40 | Example 2 | 60 | | | | | 36 | E1-11<br>E1-12 | 3.5<br>0.5 | – | ◎ | ◎ | ◎ | ◎ | ◎ | ◎ |
| Examples | 41 | Example 4 | 30 | a-3 | 10 | b1-1<br>b1-2 | 20<br>17 | 20 | E1-1 | 3 | ○ | – | ◎ | ◎ | ○ | ◎ | ◎ |
| Examples | 42 | Example 5 | 40 | | | b1-1<br>b1-2 | 20<br>17 | 18 | E1-1<br>E2-4 | 3<br>2 | ◎ | – | ◎ | ◎ | ◎ | ◎ | ◎ |
| Examples | 43 | Example 6 | 20 | | | b1-1 | 18 | 40 | E1-1<br>E2-1<br>E1-11<br>E1-12 | 2<br>17<br>2.5<br>0.5 | – | ◎ | ◎ | ◎ | ◎ | ○ | ◎ |
| Examples | 44 | Example 8 | 70 | | | b1-1 | 15 | 12 | E1-1 | 3 | ◎ | – | ○ | ◎ | ◎ | ◎ | ◎ |
| Examples | 45 | Example 11 | 60 | a-1 | 2 | b1-2 | 23 | 12 | E1-1 | 3 | ◎ | – | ◎ | ◎ | ◎ | ○ | ○ |
| Comparative Examples | 15 | Comparative Example 1 | 60 | | | b1-1 | 7 | 30 | E1-1 | 3 | × | – | ◎ | × | △ | × | × |
| Comparative Examples | 16 | Comparative Example 5 | 40 | | | b1-1<br>b1-2 | 20<br>10 | 23 | E1-1<br>E1-11<br>E1-12 | 3<br>3.5<br>0.5 | – | △ | ◎ | △ | × | △ | △ |

As is clear from the results shown in Table 6, the aqueous (water-based) ink compositions of Examples had low viscosity, high compatibility or pigment dispersibility, and good ejection stability in inkjet printing. In addition, the aqueous ink compositions of Examples had high curability while having good storage stability. The reason why such a result is obtained is that the aqueous ink compositions of Examples contain the water-insoluble polyfunctional (meth)acrylamide (A) and the polymerizable compound (B), the compatibility between (A) and (B) is extremely high, and the dispersibility and water solubility of the pigments are also excellent. It was confirmed that the printed matter obtained by curing the aqueous ink composition of Example had excellent surface dryness and print clarity. On the other hand, all the aqueous (water-based) ink compositions of Comparative Examples and the printed matters thereof did not satisfy the evaluation of characteristics in many items.

Examples 46 to 51 and Comparative Examples 17 and 18 (Preparation and Evaluation of Three-dimensional Modeling Ink Composition)

The curable composition, the polyfunctional (meth)acrylamide (A), the polymerizable compound (B), the photopolymerization initiator (C), and other components obtained in Table 1 were weighed in mass ratios shown in Table 7 and mixed at 35 °C for 1 hour to prepare the three-dimensional modeling ink compositions of Examples 46 to 51 and Comparative Examples 17 and 18. Using the obtained three-dimensional modeling ink composition, a three-dimensional modeled object was produced by the following method, and the cure shrinkage resistance of the three-dimensional modeling ink composition, and the strength, impact resistance, water resistance, and modeling accuracy of the obtained cured product were evaluated, and the evaluation results are shown in Table 7.

Production of Three-dimensional Object and Strength Evaluation

A heavy release PET film with a thickness of 75 $\mu$m (manufactured by Toyobo Co., Ltd., E7001 polyester films) was adhered onto a horizontally placed glass plate, and a spacer with a thickness of 1 mm and internal portion punched out in a No. 2 dumbbell shape in accordance with JIS K6251 was placed, the three-dimensional modeling ink compositions obtained in Examples and Comparative Examples, were filled inside the spacer, and a light release PET film (manufactured by Toyobo Co., Ltd., E7002 polyester films) with a thickness of 50 $\mu$m was further overlaid thereon, and the three-dimensional modeling ink compositions were cured by irradiation with UV rays from both sides (apparatus: inverter-type conveyor device ECS-4011GX manufactured by Eye Graphics Co., Ltd., metal halide lamp: M04-L41 manufactured by Eye Graphics Co., Ltd., UV illuminance: 200 mW/cm$^2$, integrated light quantity: 1000 mJ/cm $^2$). Thereafter, the release PET films on both sides were removed to obtain a test piece of the cured product for strength evaluation. In accordance

with JIS K7161, tensile strength was measured using a tabletop precision universal testing machine (Autograph AGS-X manufactured by Shimadzu Corporation) in a temperature environment of 25 °C under the conditions of a tensile speed of 10 mm/min and a chuck-to-chuck spacing of 50 mm, and the strength was evaluated according to the following criteria.

◎: The tensile strength was 40 MPa or more.
O: The tensile strength was 30 MPa or more and less than 40 MPa.
△: The tensile strength was 20 MPa or more and less than 30 MPa.
×: The tensile strength was less than 20 MPa.

Three-dimensional Modeling Ink Composition Cure Shrinkage Resistance Evaluation

A cured product was prepared in the same manner as the test piece for the strength evaluation described above, and the cure shrinkage rate was calculated from the specific gravity (d0) of the curable composition before curing and the specific gravity (d1) of the sheet-shaped cured product obtained after curing according to the JIS K5600 2-4 by the following formula. The cure shrinkage resistance was evaluated based on the following criteria. The lower the cure shrinkage, the higher the cure shrinkage resistance.

$$\text{Cure shrinkage (\%)} = (d1\text{-}d0)/d1 \times 100\%$$

◎: Cure shrinkage was less than 2%.
O: Cure shrinkage was 2% or more and less than 5%.
△: Cure shrinkage was 5% or more and less than 10%.
×: Cure shrinkage was 10% or more.

Evaluation of Impact Resistance (Toughness) of Three-dimensional Object

In the same manner as in the preparation of the test pieces for the strength evaluation described above, a spacer having a thickness of 4 mm and an internal dimension of 10 × 80 mm was prepared, and the inside of the spacer was filled with the ink composition for three-dimensional modeling obtained in each of the Examples and Comparative Examples to a thickness of 4 mm. A light release PET film was similarly placed on top of the spacer, and UV ray was irradiated from both sides to cure the ink composition. Thereafter the release PET films on both sides are removed, and the cured products were further irradiated with UV rays at a predetermined integrated light quantity (apparatus: desktop batch type UV-LED curing apparatus MUVBA-0.3 × 0.3 × 0.5 manufactured by ITEC CORPORATION Systems Inc., wavelength: 405 nm, illumination: (UV-V) 50 $mW/cm^2$, integrated light quantity: 5,000 $mJ/cm^2$), then post-curing was performed to Cure completely. Using the obtained cured product as a test piece, Izod impact strength (notched) was measured in accordance with JIS K 7110, and the impact resistance was evaluated as follows. An Izod-Charpy impact tester "Model No.195 -R" manufactured by Yasuda Seiki Seisakusho Ltd. was used. The higher the impact strength, the higher the impact resistance.

◎: Impact strength was 40J/m or more.
O: Impact strength was 30J/m or more and less than 40J/m.
△: Impact strength was 20J/m or more and less than 30J/m.
×: Impact strength was less than 20J/m.

Water Resistance Evaluation of Three-dimensional Object

Using a spacer having a thickness of 10 mm and an inner of 10 cm × 1 cm, a cured product test piece for water resistance evaluation having a length 10 cm, a width of 1 cm, and a thickness of 1 mm was prepared by the method of strength evaluation described above. After measuring the weight of the obtained test piece immediately after shaping, the test piece was immersed in a beaker containing 100 ml of water, and the weight after immersion was measured after one day. The water absorption was measured by substituting the weight before immersion and the weight after immersion into the following formula, and the water resistance was evaluated according to the following criteria. The lower the water absorption rate, the higher the water resistance.

Water absorption rate (%) = (weight after immersion - weight before immersion) / weight before immersion × 100%

◎: Water absorption rate was less than 2%.
O: Water absorption rate was 2% or more and less than 2.5%.
△: Water absorption rate was 2.5% or more and less than 3%.
×: Water absorption rate was 3% or more.

Modeling Accuracy Evaluation

In the same manner as in the preparation of the test pieces for the strength evaluation, a spacer having a thickness of 10 mm and an internal area of 10 × 10 mm was prepared, and the ink composition for three-dimensional modeling obtained in each of the Examples and Comparative Examples was filled with a thickness of 1 mm inside the spacer, and then the surface was smoothed by keeping the ink composition at 60 ° C. for 30 seconds, and then the ink composition for three-dimensional modeling was similarly irradiated with ultraviolet light to cure the ink composition for three-dimensional modeling. The ink composition for three-dimensional modeling was then filled to a thickness of 1 mm on the obtained cured product, and the curing process was repeated 10 times to obtain a cured product of 10 × 10 × 10 mm. The height of the obtained cured product was measured, and the side surface was visually observed, and the modeling accuracy was evaluated according to the following criteria.

◎: The height was less than 10 mm ± 0.1 mm, and there was no unevenness on the side surface.
O: The height was 10 mm ± 0.1 mm or more and less than ± 0.2 mm, or there was slight unevenness on the side surface.
△: The height was 10 mm ± 0.2 mm or more and less than ± 0.3 mm, or there was some unevenness on the side surface.
×: The height was 10 mm is ± 0.3 mm or more, or there was obvious unevenness on the side surface.

[Table 7]

| | | Ink Composition for Three-dimensional Modeling | | | | | | | | Properties of Three-dimensional Object | | | | |
|---|---|---|---|---|---|---|---|---|---|---|---|---|---|---|
| | | Curable Composition | | A | | B | | Others | | Strength | Cure Shrinkage Resistance | Impact Resistance | Water Resistance | Modeling Accuracy |
| | | Type | Mass (%) | Type | Mass (%) | Type | Mass (%) | Type | Mass (%) | | | | | |
| Examples | 46 | Example 3 | 50 | | | b1-18<br>b2-1 | 30<br>15 | c-1 | 5 | ◎ | ◎ | ◎ | ◎ | ◎ |
| | 47 | Example 2<br>Example 10 | 40<br>30 | | | b1-12<br>b2-2 | 18<br>12 | | | ◎ | ◎ | ◎ | ○ | ◎ |
| | 48 | Example 8 | 15 | a-1 | 35 | b1-1<br>b2-2 | 30<br>15 | c-2 | 5 | ◎ | ◎ | ◎ | ◎ | ◎ |
| | 49 | Example 14 | 35 | | | b1-1<br>b1-10<br>b2-7 | 22<br>20<br>20 | E1-2 | 3 | ◎ | ◎ | ◎ | ◎ | ◎ |
| | 50 | Example 13 | 35 | | | b1-2<br>b1-4<br>b2-2 | 30<br>10<br>20 | c-4 | 5 | ◎ | ◎ | ◎ | ○ | ◎ |
| | 51 | Example 6 | 90 | | | b1-1 | 10 | | | ◎ | ◎ | ◎ | ◎ | ◎ |
| Comparative Examples | 17 | Comparative Example 3 | 50 | | | b1-13<br>b2-2 | 20<br>25 | E1-1 | 5 | △ | × | × | ○ | × |
| | 18 | Comparative Example 5 | 75 | | | b1-14<br>b2-5 | 10<br>10 | E1-2 | 5 | × | △ | △ | × | × |

As is clear from the results of Table 7, the ink compositions for three-dimensional modeling of Examples containing the water-insoluble polyfunctional (meth)acrylamide (A) and the polymerizable compound (B), the strength, the impact resistance, the water resistance, and the modeling accuracy of the obtained molded objects were all favorable. In

Examples 49 and 51, which contained (A) having a branched isopropyleneoxy group, further improvements in the cure shrinkage resistance of the ink composition for three-dimensional modeling and the water resistance and impact resistance of the resulting model were confirmed. On the other hand, in Comparative Examples in which (A) and (B) were not simultaneously contained, the cure shrinkage resistance of the three-dimensional modeling ink composition was low, and various properties of the obtained molded objects were also low.

Examples 52 to 57 and Comparative Examples 19 and 20 (Preparation and Evaluation of water-based Coating Composition)

The curable composition obtained in Table 1, the polyfunctional (meth)acrylamide (A), the polymerizable compound (B), the photopolymerization initiator (C), and other components were weighed in mass ratios shown in Table 8, and mixed at 35 °C for 1 hour to prepare the aqueous (water-based) coating compositions of Examples 52 to 57 and Comparative Examples 19 and 20. The obtained water-based coating composition was applied onto an ABS substrate with a bar coater (No. 12) (thickness after drying: 10 μm), and dried with a hot air dryer for 10 minutes (80 °C). The dried coating films obtained in Examples 52 to 56 and Comparative Examples 19 and 20 were cured by irradiation with UV rays (the same inverter-type conveyor device manufactured by Eye Graphics Co., Ltd. as described above, the same metal halide lamp as described above, ultraviolet irradiance: 300 mW/cm$^2$, integrated light quantity: 2000 mJ/cm$^2$) to obtain UV-cured products (cured coating films). The dried coating film obtained in Example 57 was heated and cured for 10 minutes by a hot-air dryer in a 120 °C to obtain a thermally cured product (cured coating film). The appearance, adhesion, hot water resistance and chemical resistance of the obtained cured product were evaluated by the following methods, and the results are shown in Table 8.

Appearance Evaluation of Cured Water-based Paint Coating Films

The obtained cured coating films was visually observed, and their appearance were evaluated by the surface smoothness and transparency of the cured coating films according to the following criteria.

Coating film appearance:

◎: The surface was smooth, and the coating film was transparent.
O: The surface was smooth, and the coating film was transparent overall with slight cloudy areas.
△: The surface was uneven or the coating film had cloudy areas.
×: The surface was uneven, and the coating film was cloudy.

Evaluation of Adhesion of Cured Water-based Paint Coating Films

Using the obtained pressure-sensitive adhesive sheet, 100 squares with 1 mm$^2$ were cross-cut with a cutter knife in accordance with JIS K 5600, a cellophane tape was attached, and the number of squares in which the pressure-sensitive adhesive layer remained on the substrate side when the tape was peeled off at once was counted, and the adhesion was evaluated according to the following criteria.

◎: 100 pieces without peeling
○: 95 to 99 pieces without peeling
△: 70 to 94 pieces without peeling
×: 0 to 69 pieces without peeling
◎: The number of remaining squares was100.
○: The number of remaining squares was 90 or more and less than 100.
△: The number of remaining squares was 50 or more and less than 90.
×: The number of remaining squares was less than 50.

Hot Water Resistance Evaluation of Cured Water-based Paint Coating Films

The obtained cured film on the ABS substrate was immersed in warm water at 80 °C for 2 hours together with the ABS plate, then taken out and dried on 25 °C for 2 hours. Thereafter, the adhesion evaluation described above was performed, the number of remaining squares was counted, and the hot water resistance was evaluated according to the following criteria. The larger the number of remaining squares, the higher the hot water resistance of.

◎: The number of remaining squares was100.
O: The number of remaining squares was 90 or more and less than 100.
△: The number of remaining squares 50 or more and less than 90.
×: The number of remaining squares was less than 50.

Chemical Resistance Evaluation of Cured Water-based Paint Coating Films

The obtained cured film on the ABS substrate was immersed together with the ABS plate in NaOH aqueous solution at pH9 with 60 °C for 1 hour, and then taken out and dried with 25 °C for 2 hours. Thereafter, the adhesion evaluation described above was performed, the number of remaining squares was counted, and the chemical resistance was evaluated according to the following criteria. The larger the number of remaining squares, the higher the chemical resistance.

◎: The number of remaining squares was100.
O: The number of remaining squares was 90 or more and less than 100.
△: The number of remaining squares was 50 or more and less than 90.
×: The number of remaining squares was less than 50.

[Table 8]

| | | Aqueous Coating Composition | | | | | | | | | Physical properties of coating film | | | |
|---|---|---|---|---|---|---|---|---|---|---|---|---|---|---|
| | | Curable Composition | | A | | B | | water | Others | | Appearance | Adhesion | Hot Water Resistance | Chemical Resistance |
| | | Type | Mass (%) | Type | Mass (%) | Type | Mass (%) | Mass (%) | Type | Mass (%) | | | | |
| Examples | 52 | Example 1 | 30 | | | b1-2 | 7 | 60 | E1-1<br>E1-8 | 2.8<br>0.2 | ◎ | ◎ | ◎ | ◎ |
| | 53 | Example 2 | 40 | | | b1-1 | 10 | 48 | E1-1 | 2 | ◎ | ◎ | ◎ | ◎ |
| | 54 | Example 6 | 10 | a-4 | 10 | b1-1 | 6 | 70 | E1-1<br>E1-9 | 2<br>2 | ◎ | ◎ | O | O |
| | 55 | Example 14 | 40 | | | b1-2<br>b1-3 | 6<br>20 | 30 | E1-1 | 4 | ◎ | ◎ | O | ◎ |
| | 56 | Example 3 | 48 | | | | | 50 | E1-1 | 2 | ◎ | ◎ | ◎ | ◎ |
| | 57 | Example 15 | 35 | a-5 | 5 | b1-1 | 17 | 40 | E1-10 | 3 | ◎ | ◎ | O | ◎ |
| Comparative Examples | 19 | Comparative Example 1 | 30 | | | b1-1<br>b1-6 | 20<br>7 | 40 | E1-1 | 3 | △ | × | × | × |
| | 20 | Comparative Example 6 | 45 | | | b1-5 | 3 | 50 | E1-1<br>E1-8 | 1.8<br>0.2 | × | × | △ | △ |

As is clear from the results shown in Table 8, the water-based coating compositions of Examples had high adhesion, could be cured by UV (active energy ray) or heat, and could obtain cured coating films having high transparency and high surface smoothness, that is, excellent appearance. The obtained cured coating film had good hot water resistance and chemical resistance. As described above, these results were obtained because the water-based coating compositions of Examples contained both the water-insoluble polyfunctional (meth)acrylamide (A) and the polymerizable compound (B). On the other hand, the water-based coating compositions of Comparative Examples did not contain (A) and (B) at the same time, so that the properties of the cured coating films were not satisfactory.

Examples 58 to 62 and Comparative Examples 21 and 22 (Preparation and Evaluation of Sealant Composition)

The curable composition obtained in Table 1, the polyfunctional (meth)acrylamide (A), the polymerizable compound (B), the photopolymerization initiator (C), and other components were weighed in mass ratios shown in Table 9, and mixed at 35 °C for 1 hour to prepare the sealant compositions of Examples 58 to 62 and Comparative Examples 21 and 22. Using the obtained sealing agent composition, a sealing agent cured product (sealing layer) was produced by the following method, and the properties of the obtained sealing layer were evaluated as follows.

Preparation of Sealant Cured Product (Sealing Layer)

A silicone spacer (vertical 50 mm, horizontal 50 mm and thick 5 mm) was set on a glass plate (vertical 30 mm, horizontal 15 mm and thick 3 mm), a copper foil (vertical 5 mm, horizontal 5 m and thick 80 μm) was placed inside the spacer, and the prepared curable sealant composition was injected. After sufficient degassing, the sealant was irradiated with UV rays (the same inverter-type conveyor device manufactured by Eye Graphics Co., Ltd. described above, the same metal halide lamp described above, UV ray illumination: 700 $mW/cm^2$, integrated light quantity: 1000 $mJ/cm^2$) to obtain a cured sealant. The properties of the obtained cured product were evaluated by the following methods, and the results are shown in Table 9.

Transparency Evaluation of Cured Sealant

A portion where the copper foil was not inserted was cut out from the obtained sealing agent cured product, and after allowing to stand for 24 hour under an atmosphere of a temperature of 23 °C and a relative humidity of 50%, the transmittance of the cured product was measured using a haze meter (NDH-2000, manufactured by Nippon Denshoku Industries Co., Ltd), and the transparency was evaluated according to the following criteria. The higher the transmittance, the higher the transparency.

◎: The transmissivity was 90% or more.
O: Transmissivity was 85% or more and less than 90%.
△: Transmissivity was 50% or more and less than 85%.
×: Transmissivity was less than 50%.

Water Resistance Evaluation of Cured Sealant

1 g of the obtained cured product was cut out and set as a test piece in a thermo-hygrostat at a temperature of 85°C and a relative humidity of 95%. After standing for 48 hours, the test piece was weighed again, the water absorption rate was calculated by the following formula, and the water resistance was evaluated by the following criteria. The lower the water absorption, the higher the water resistance.

Water absorption rate (%) = (weight after water-weight - before water absorption)/weight before water absorption × 100%

◎: Water absorption was less than 1.0%.
O: Water absorption rate was 1.0% or more and less than 2.0%.
△: Water absorption rate was 2.0% or more and less than 3.0%.
×: Water absorption rate was 3.0% or more.

Outgassing Resistance Evaluation

1 g of the obtained cured product was cut out and left to stand as a test piece in a thermostatic chamber set at a temperature of 100°C, a dry nitrogen stream was allowed to flow for 24 hours, the weight of the test piece was measured again, the generation rate of outgassing was calculated by the following formula, and evaluation was performed in four grades. The lower the generation rate of outgas, the higher the outgas resistance.

Outgassing generation rate (%) = (weight after test - weight before test) / weight before test × 100%

◎: Outgassing generation rate was less than 0.1%.
O: Outgassing generation rate was 0.1% or more and less than 0.3%.
△: Outgassing generation rate was 0.3% or more and less than 1.0%.
×: Outgassing generation rate was 1.0% or more.

Moist-heat Yellowing Resistance Evaluation

The obtained cured product of the sealing agent was allowed to stand for 24 hours in an atmosphere at a temperature of 23 °C and a relative humidity of 50%, and then the transmission spectrum of the cured product was measured with a dedicated transmission color measuring machine (TZ-6000, manufactured by Nippon Denshoku Industries Co., Ltd) to obtain an initial b-value. Thereafter, the cured product was allowed to stand for 500 hours in a thermo-hygrostat set at 85 °C and a relative humidity of 85%, and an accelerated test for moist-heat yellowing resistance was performed. The cured product after the test was similarly allowed to stand under an atmosphere at a temperature of 23 °C and a relative humidity of 50% for 24 hours, and the transmitted color was measured to obtain a b-value after moist-heat test. The difference between the b value after test and the initial b-value was defined as a change value Δb (Δb = b-value after moist-heat test - initial b-value). The moist-heat yellowing resistance of the cured product was evaluated according to the following criteria.

◎: Both the initial b-value and the b-value after moist-heat test were 0.2 or less, and Δb was 0.1 or less.
O: Either one or both of the initial b-value and the b-value after moist-heat test was 0.2 or more, but both were 0.5 or less, and Δb was 0.2 or less.
Δ: Either one or both of the initial b-value and the b-value after moist-heat test were 0.5 or more, but both were 1.0 or less, and Δb was 0.3 or less.
×: Either one or both of the initial b-value and the b-value after moist-heat test were 1.0 or more, or Δb was 0.3 or more.

Heat Cycle Resistance Evaluation

The obtained sealant cured product was allowed to stand at -40 °C for 30 minutes and then at 100 °C for 30 minutes as one cycle, and this cycle was repeated 100 times. The state of the cured product was visually observed, and the heat cycle resistance was evaluated according to the following criteria.

◎: No change was observed.
O: Slight bubble generation was observed, but crack generation was not observed. The cured product was transparent.
△: Some bubbles or cracks were observed and the cured product was slightly cloudy.
×: Bubbles or cracks occur over the entire surface and the cured product was in a translucent state.

Corrosion Resistance Evaluation

After the moist-heat yellowing resistance test, the surface of the copper foil was visually observed, and the corrosion resistance of the cured product was evaluated in four grades.

◎: No corrosion was observed.
O: Slight corrosion was observed.
△: Some corrosion was observed.
×: Significant corrosion was observed.

[Table 9]

| | | Sealant Composition | | | | | | | | Transparency | Water Resistance | Outgassing Resistance | Moist-heat Yellowing Resistance | Heat Cycle Resistance | Corrosion Resistance |
|---|---|---|---|---|---|---|---|---|---|---|---|---|---|---|---|
| | | Curable Composition | | A | | B | | Others | | | | | | | |
| | | Type | Mass (%) | Type | Mass (%) | Type | Mass (%) | Type | Mass (%) | | | | | | |
| Examples | 58 | Example 3 | 50 | | | b1-12<br>b2-3 | 30<br>15 | c-1 | 5 | ◎ | ◎ | ◎ | ◎ | ◎ | ◎ |
| | 59 | Example 4 | 70 | | | b1-12<br>b2-2 | 20<br>5 | c-3 | 5 | ◎ | ◎ | ○ | ◎ | ◎ | ◎ |
| | 60 | Example 5 | 50 | a-2 | 10 | b1-20<br>b2-2 | 15<br>20 | c-2 | 5 | ◎ | ◎ | ◎ | ◎ | ◎ | ◎ |
| | 61 | Example 8 | 60 | a-6 | 10 | b1-1<br>b2-1 | 12<br>15 | E1-3 | 3 | ◎ | ◎ | ◎ | ◎ | ◎ | ◎ |
| | 62 | Example 15 | 100 | | | | | | | ◎ | ○ | ○ | ○ | ◎ | ◎ |
| Comparative Examples | 21 | Comparative Example 3 | 50 | | | b1-13<br>b2-2 | 20<br>25 | c-1 | 5 | × | △ | × | △ | △ | ◎ |
| | 22 | Comparative Example 5 | 75 | | | b1-14<br>b2-5 | 10<br>10 | c-2 | 5 | × | × | × | △ | △ | ◎ |

As is clear from the results shown in Table 9, the sealant compositions of Examples contained the water-insoluble polyfunctional (meth)acrylamide (A) and the polymerizable compound (B), the obtained sealant cured products had high water resistance, generated less outgassing, and were good in all of moist-heat yellowing resistance, heat cycle resistance, and corrosion resistance due to a having many isopropyleneoxy groups of (meth)acrylamide of (A). In addition, since the compatibility between (A) and (B) was high, the transparency of the sealant cured product was also high. On the other hand, the cured products obtained from the curable compositions of Comparative Examples did not satisfy any of these properties. The sealant composition of the present disclosure can be suitably used as a sealant for an optical member, an electrical device, or the like.

Examples 63 to 67 and Comparative Examples 23 and 24 (Preparation and Evaluation of Curable Nail Cosmetic)

The curable composition obtained in Table 1, the (meth)acrylamide (A), the polymerizable compound (B), the photopolymerization initiator (C), and other components were weighed in mass ratios shown in Table 10, and mixed at 35 °C for 1 hour to prepare a nail cosmetic of each of Examples and Comparative Examples. Using the obtained nail cosmetic, curability and properties of the obtained cured film were evaluated by the following methods, and the results are shown in Table 10.

Nail Cosmetic Curability Evaluation

The obtained curable nail cosmetic is applied on a nylon 6 test piece ("SHT-N6 (NC)" manufactured by Toray plastic Seiko Co., Ltd) using a bar coater (No. 12) so as to have a film with thickness of 10 μm, and then irradiated with UV rays using a UV-LED lamp dedicated to gel nails (manufactured by Beauty Nailer Co., Ltd., wavelengths: 405 nm, 48 W), the time was measured until the tack disappeared when the surface of the cured film was touched, and the curability of the nail cosmetic was evaluated according to the following criteria. The shorter the time required to eliminate the tack, the higher the curability.

◎: Tack disappeared in less than 1 minute.
O: Tack disappeared in 1 minute or more and less than 3 minutes.
△: Tack disappeared in 3 minutes or more and less than 10 minutes.
×: The tack did not disappear even after 10 minutes or more.

Adhesion Evaluation of Nail Cosmetic

The obtained curable nail cosmetic was applied onto a nylon 6 test piece in the same manner described above, and irradiated with light for 3 minutes to form a cured film. On the surface of the obtained cured films 100 squares with 1

$mm^2$ were crosscut with a cutter knife in accordance with JIS K 5600, and the number of squares remaining on the test piece when a commercially available cellophane tape was bonded and then peeled off was evaluated in four grades. The greater the number of squares remaining on the test piece, the higher the adhesion.

◎: The number of remaining squares was 100.
O: The number of remaining squares was 90 to 99.
△: The number of remaining squares was 60 to 89.
×: The number of remaining squares was less than 60.

Surface Hardness Evaluation of Nail Cosmetic Cured Film

The cured film of each of Examples and Comparative Examples was produced in the same manner as in the adhesion evaluation, the surface of the obtained film was pulled with a pencil having a hardness of HB under a load of 750 g, the presence or absence of peeling and the presence or absence of scratches were visually observed, and the surface hardness of the nail cosmetic cured film was evaluated according to the following criteria. The less the occurrence of scratches and peeling, the higher the surface hardness.

O: Neither scratches nor peeling occurred. The surface hardness was pencil hardness HB or more.
△: Peeling did not occur, but scratches occurred.
×: Peeling occurred.

Surface Glossiness Evaluation of Nail Cosmetic Cured Film

Cured films of Examples and Comparative Examples were prepared in the same manner as in the adhesion evaluation, and the surface gloss of the cured film was visually observed and evaluated according to the following criteria.

O: The surface was glossy.
△: Reflection of light could be confirmed, while cloudiness was observed.
×: No reflection of light was observed, and there was no gloss.

[Table 10]

| | | Nail Cosmetic Composition | | | | | | | | Curability | Adhesion | Surface Hardness | Surface Glossiness |
|---|---|---|---|---|---|---|---|---|---|---|---|---|---|
| | | Curable Composition | | A | | B | | Others | | | | | |
| | | Type | Mass (%) | Type | Mass (%) | Type | Mass (%) | Type | Mass (%) | | | | |
| Examples | 63 | Example 2 | 53 | | | b1-1 | 12 | c-1 | 5 | ◎ | ◎ | ○ | ○ |
| | | | | | | b1-3 | 15 | | | | | | |
| | | | | | | b2-7 | 15 | | | | | | |
| | 64 | Example 3 | 50 | | | b1-3 | 15 | c-4 | 10 | ◎ | ◎ | ○ | ○ |
| | | | | | | b2-1 | 25 | | | | | | |
| | 65 | Example 6 | 30 | a-4 | 15 | b1-1 | 15 | c-2 | 5 | ◎ | ◎ | ○ | ○ |
| | | | | | | b1-11 | 15 | | | | | | |
| | | | | | | b2-2 | 20 | | | | | | |
| | 66 | Example 14 | 80 | | | b1-2 | 17 | E1-3 | 3 | ◎ | ◎ | △ | ○ |
| | 67 | Example 7 | 83 | | | b1-2 | 15 | E1-2 | 2 | ◎ | ○ | ○ | △ |
| Comparative Examples | 23 | Comparative Example 1 | 80 | | | b1-2 | 15 | c-1 | 5 | × | △ | × | × |
| | 24 | Comparative Example 5 | 70 | | | b1-1 | 15 | c-2 | 5 | △ | × | × | × |
| | | | | | | b1-8 | 10 | | | | | | |

As is clear from the results in Table 10, in the performance evaluation using a commercially available UV lamp dedicated to gel nails, the curable nail cosmetics of Examples containing the (meth)acrylamide (A) and the polymerizable compound (B) at the same time, had excellent curability and high adhesion to a nylon base material (a material having many amide groups as in nails containing proteins as a main component). From these results, it was found that the curable nail cosmetic of the present disclosure can be suitably used as a gel nail for base coating to be directly applied to a nail. Further, the obtained cured film has good surface hardness and surface glossiness, and can be suitably used as a gel nail for top coating. On the other hand, the curable compositions of Comparative Examples had low curability, and the surface hardness and surface glossiness of the cured film were low.

Examples 68 to 72 and Comparative Examples 25 and 26 (Preparation and Evaluation of Curable Dental Materials)

The curable composition obtained in Table 1, the polyfunctional (meth)acrylamide (A), the polymerizable compound (B), the photopolymerization initiator (C) and other components were weighed in mass ratios shown in Table 11, and mixed at 35 °C for 1 hour to prepare curable dental materials of Examples and Comparative Examples. The solubility or dispersibility (when an insoluble inorganic filler was blended) of the obtained curable dental material was visually observed. The curable dental material was used to prepare a dental material cured product by the following method, and the curability of the curable dental material and the surface smoothness, hardness, and adhesive strength of the obtained dental material cured product were evaluated. The evaluation results are shown in Table 11.

Solubility or Dispersibility Evaluation of Curable Composition for Dental Material

◎: The obtained composition was uniform and transparent.
O: The obtained composition was uniform and translucent.
△: The resulting composition was cloudy, and it was difficult to judge homogeneity.

×: The resulting composition was completely immiscible.

Curability Evaluation of Curable Composition for Dental Material

Using each of the obtained curable composition of Examples and Comparative Examples, a mold (20 mm × 20 mm × 10 mm) made of polytetrafluoroethylene having a hole with a diameter of 6 mm at the center was filled with the composition, pressed with a polypropylene film, and irradiated for 30 seconds with a dental light irradiator (Tokuso Power light, manufactured by Tokuyama Dental Co., Ltd., light power density 700 mW/cm$^2$, light intensity on irradiated surface 640 to 650 mW/cm$^2$, light sources are halogen lamps, irradiation aperture 8 mm) in close contact with the polypropylene film. The polypropylene film was peeled off and the cured product was touched by hand to confirm stickiness and the presence or absence of an uncured component.

◎: There was no stickiness (complete curing).
O: There was some stickiness, but no finger marks remained on the surface (approximately fully cured, no wiping of uncured ingredients was necessary).
△: There was stickiness, and finger marks remained on the surface (incomplete curing, necessitating wiping of the uncured ingredients).
×: Stickiness was severe, and finger sticked to the surface (many uncured components remained and could not be used as a cured film).

Surface Smoothness Evaluation of Curable Composition for Dental Material

The surface of the cured product obtained in the curability evaluation was visually observed to confirm smoothness and glossiness, and the surface smoothness was evaluated according to the following criteria.

◎: The surface was smooth and glossy.
O: The surface was almost smooth with smooth haze or slight irregularities.
△: The entire surface was cloudy, and some irregularities or grains were observed.
×: The surface was entirely cloudy and covered with granules.

Hardness Evaluation of Curable Composition for Dental Material

The surface of the cured product obtained in the curability evaluation was buffed and the Knoop hardness was measured with a microhardness meter manufactured by Matsumoto Seiki Co., Ltd. at a load of 10 g for 20 seconds. The measurement temperature was 23°C. The higher the Knoop hardness, the higher the hardness of the cured composition.

◎: Knoop hardness was 200 KHN or more (corresponding to permanent tooth enamel).
O: Knoop hardness was 70 KHN or more and less than 200 KHN (corresponding to dentin).
△: Knoop hardness was less than 70 KHN.
×: The composition did not cure, so hardness measurements could not be made.

Adhesive Strength (dentin adhesive force) Evaluation of Curable Composition for Dental Material

The forehead of a bovine forehead was ground with #1000 water-resistant abrasive paper under pouring water, a flat bonding dentin surface was scraped off, and dried by blowing compressed air for 10 seconds, and a tape having a hole with a diameter of 3 mm was attached thereto to set an adherend surface. Thereafter, an adhesive test piece was prepared by a known method (see the method described in JP2010-208964A). The adhesion test piece was immersed in water at 37°C for 24 hours, and then the tensile adhesion strength was measured using an Instron universal tester (crosshead speed: 2 mm/min), and the adhesion strength to enamel and dentin of the curable compositions obtained in Examples and Comparative Examples was determined. The value of the tensile adhesion strength was an average value of five test pieces.

◎: Adhesion strength between enamel and dentin was 20 Mpa or more.
O: Adhesion strength between the enamel and the dentin was 20 MPa or more.
△: Adhesion strength between enamel and dentin was 7 Mpa or more.
×: Adhesion strength between enamel and dentin was less than 7 Mpa.

[Table 11]

| | | Composition for Dental Material | | | | | | | | Solubility or Dispersibility | Curability | Surface Smoothness | Hardness | Adhesive Strength |
|---|---|---|---|---|---|---|---|---|---|---|---|---|---|---|
| | | Curable Composition | | A | | B | | Others | | | | | | |
| | | Type | Mass (%) | Type | Mass (%) | Type | Mass (%) | Type | Mass (%) | | | | | |
| Examples | 68 | Example 3 | 55 | | | b1-1<br>b2-1 | 20<br>18 | E1-2<br>E1-13 | 3<br>4 | ◎ | ◎ | ◎ | ◎ | ◎ |
| | 69 | Example 6 | 50 | a-4 | 5 | b1-1<br>b2-1 | 25<br>10 | c-4 | 10 | ◎ | ◎ | ◎ | ◎ | ◎ |
| | 70 | Example 8 | 30 | | | b1-1<br>b1-11<br>b2-2 | 15<br>20<br>30 | c-2 | 5 | ◎ | ◎ | ◎ | ◎ | ◎ |
| | 71 | Example 5 | 27 | | | b1-2<br>b2-4 | 40<br>25 | E1-3<br>E1-13 | 3<br>5 | ◎ | ◎ | ◎ | ◎ | ○ |
| | 72 | Example 2 | 10 | | | b1-1<br>b2-1<br>b2-5 | 40<br>30<br>10 | c-4 | 10 | ◎ | ◎ | ○ | ◎ | ○ |
| Comparative Examples | 25 | Comparative Example 1 | 50 | | | b1-2<br>b2-1 | 30<br>15 | c-1 | 5 | △ | △ | × | × | × |
| | 26 | Comparative Example 6 | 80 | | | b2-2 | 13 | c-2<br>E1-13 | 3<br>4 | × | △ | × | △ | △ |

As is clear from the results in Table 11, the curable compositions for dental materials of Examples simultaneously contained the (meth)acrylamide (A) and the polymerizable compound (B), had excellent solubility or dispersibility, and had high curability. The cured compositions for dental materials obtained in the Examples had good hardness, surface smoothness and adhesive strength. On the other hand, the curable compositions for dental materials of Comparative Examples did not contain (A) and (B) at the same time, the solubility or dispersibility was low, sufficient curing could not proceed, the hardness and surface smoothness of the obtained cured product were low, and the adhesive strength was insufficient.

Examples 73 to 77 and Comparative Examples 27 and 28 (Preparation and Evaluation of Curable Decorative Coating Agent)

The curable composition obtained in Table 1, the polyfunctional (meth)acrylamide (A), the polymerizable compound (B), the photopolymerization initiator (C), and other components were weighed in mass ratios shown in Table 12, and mixed at 35 °C for 1 hour to prepare curable decorative coating agents of Examples and Comparative Examples. Using the obtained decorative coating agent, a laminate was produced by the following decorative processing, and the properties of the laminate were evaluated.

Laminate Production

Using a bar coater (No. 6), the obtained decorative coating agent was applied to a polycarbonate film with a thickness of 180 μm ("Panlite PC-200", manufactured by Teijin Ltd) so as to have a thickness of 5 μm after drying, heated at 80 °C for 3 minutes, and cured by UV irradiation (the same inverter-type conveyor apparatus manufactured by Eye Graphics Co., Ltd., the same metal halide lamp, UV irradiance: 700 mW/$cm^2$, integrated light quantity: 1000 mJ/$cm^2$) to obtain a laminate having a hard coat layer. The obtained laminate was cut out, and the surface tackiness resistance, surface hardness, and scratch resistance of the hard coat layer of the laminate, and the elongation rate and bending resistance of the laminate were evaluated by the following methods, and the results are shown in Table 12.

Surface Tackiness Resistance Evaluation of Hard Coat Layer of Laminate

Using the obtained laminate, the surface of the hard coat layer was touched with a finger, and the degree of stickiness was evaluated.

◎: There was no stickiness.
O: Slight stickiness was observed, but no finger mark was left on the surface.
△: It was sticky and leaved finger marks on the surface.
×: Stickiness was severe, and fingers stick to the surface.

Elongation Rating of the Laminate

The obtained laminate was cut into lengths 50 mm and widths 15 mm, fixed to a Tensilon universal tester RTA-100 (manufactured by Orientec Co., Ltd) at distances 25 mm between chucks, and pulled in one direction at a speed of 250 mm/min in an oven set at a temperature of 150 °C while visually observing the appearance. The elongation rate was calculated by the following method and evaluated according to the following criteria.

$$\text{Elongation rate (\%)} = (\text{Specimen length after test} / 25) \times 100\%$$

◎: Elongation rate was 200% or more.
O: Elongation rate was 150% or more and less than 200%.
△: Elongation percentage was 110% or more and less than 150%.
×: Elongation was less than 110%.

Surface Hardness Evaluation of Hard Coat Layer of Laminate

The hard coat layer of the laminate was scratched with pencils at an angle of 45 ° by about 10 mm in accordance with JIS K 5600, and then a hardest pencil having no scratches on the surface of the laminate was used as a pencil hardness, and the surface hardness was evaluated as follows.

◎: The pencil hardness was 2H or more.
O: The pencil hardness was HB to H.
△: The pencil hardness was 3B to B.
×: The pencil hardness was 4B or less.

Scratch Resistance Evaluation of Hard Coat Layer of Laminate

The hard coat layer of the laminate was reciprocated 10 times with a steel wool of #0000 under a load of 200 g, and the surfaces of the hard coat layers were visually observed to evaluate the scratch resistance according to the following criteria.

◎: No peeling or scratching of the film was observed.
O: Slight fine scratches were observed in a part of the film.
△: Streaky scratches were observed throughout the film.
×: Peeling of the film occurred.

Bending Resistance Evaluation of Laminate

The test piece of the laminate was bent at an angle of 180 ° so that the hard coat face was on the outside, placed on a 1 kg weight and allowed to stand for 10 minutes. The presence or absence of cracks on the surface of the laminate was visually observed and the bending resistance was evaluated as follows.

◎: No cracks were observed.
O: The folded portion was partially whitened.
△: Partial cracking was observed in the folded portion.
×: Cracks were observed in the folded portion.

[Table 12]

| | | Composition for Decorative Coating Aagent | | | | | | | | Surface Tackiness Resistance | Elongation Rating | Pencil Hardness (Surface Hardness) | Scratch Resistance | Bending Resistance |
|---|---|---|---|---|---|---|---|---|---|---|---|---|---|---|
| | | Curable Composition | | A | | B | | Others | | | | | | |
| | | Type | Mass (%) | Type | Mass (%) | Type | Mass (%) | Type | Mass (%) | | | | | |
| Examples | 73 | Example 3 | 65 | | | B2-2<br>B2-6 | 15<br>18 | E1-2 | 2 | ◎ | ◎ | ◎ | ◎ | ◎ |
| | 74 | Example 4 | 80 | A-2 | 5 | B2-1 | 13 | E1-3 | 2 | ◎ | ◎ | ◎ | ◎ | ◎ |
| | 75 | Example 5 | 25 | | | B1-1<br>B2-4<br>B2-5 | 30<br>15<br>20 | C-4 | 10 | ◎ | ◎ | ○ | ○ | ○ |
| | 76 | Example 6 | 50 | | | B1-4<br>B2-4 | 25<br>22 | E1-3 | 3 | ◎ | ◎ | ◎ | ◎ | ◎ |
| | 77 | Example 14 | 40 | | | B1-1<br>B2-1 | 40<br>15 | C-3 | 5 | ◎ | ◎ | ○ | ○ | ◎ |
| Comparative Examples | 27 | Comparative Example 3 | 50 | F-1 | 30 | B1-1<br>B2-1 | 10<br>5 | C-1 | 5 | × | △ | × | × | △ |
| | 28 | Comparative Example 5 | 80 | | | B2-2 | 20 | | | ○ | × | △ | △ | × |

As is clear from the results of Table 12, the curable decorative coating agents of the Examples contained the polyfunctional (meth)acrylamide (A) and the polymerizable compound (B), and it was possible to easily obtain a laminate having a decorative coating layer (decorative coating film) by coating and curing the curable decorative coating agents on the surfaces of general-purpose plastic substrates. The surface (coated with a hard coat agent or the like) of the obtained laminate had tackiness resistance and high hardness and scratch resistance, and the bending resistance of the obtained laminate was good. On the other hand, similar decorative performance was not confirmed for the curable decorative coating agents of Comparative Examples. The decorative coating agent of the present disclosure is suitably used for various decorative molding, decorative processing, and decorative printing such as decorative film, decorative sheet, and decorative coating.

The present disclosure includes the following contents.

(1) A curable composition comprises one or more water-insoluble polyfunctional (meth)acrylamide (A) and one or more polymerizable compound (B) other than (A).
(2) The curable composition according to the above (1), wherein the solubility parameter (SP value) of the polyfunctional (meth)acrylamide (A) is 8.8 to 11.0 $(cal/cm^3)^{1/2}$.
(3) The curable composition according to (1) or (2) above, wherein the polymerizable compound (B) has a solubility parameter (SP value) of 8.5 to 14.5 $(cal/cm^3)^{1/2}$.
(4) The curable composition according to any one of the above (1) to (3), wherein the absolute value of the difference between the solubility parameter (SP value) of the polyfunctional (meth)acrylamide (A) and the solubility parameter (SP value) of the polymerizable compound (B) is 3.0 $(cal/cm^3)^{1/2}$ or less.
(5) The curable composition according to any one of (1) to (4) above, wherein the polyfunctional (meth)acrylamide (A) has an acrylic equivalent of 180 or more.
(6) The curable composition according to any one of the above (1) to (5), wherein the polyfunctional (meth)acrylamide (A) is a compound represented by the general formulas (1) to (4).

[Chemical Formula 1]

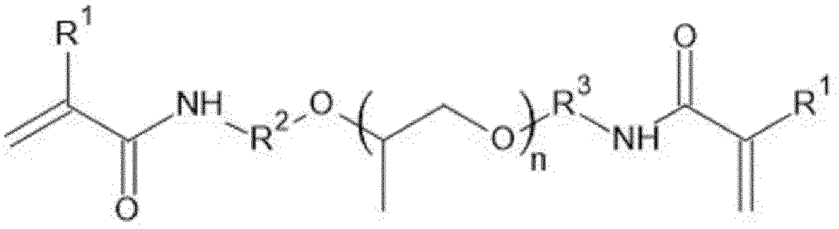

General Formula (1)

[Chemical Formula 2]

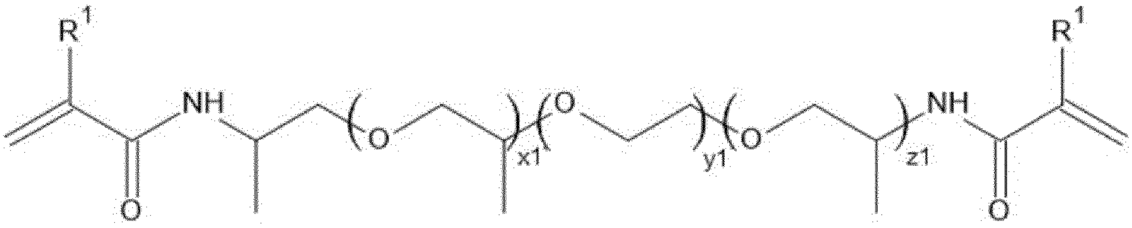

General Formula (2)

[Chemical Formula 3]

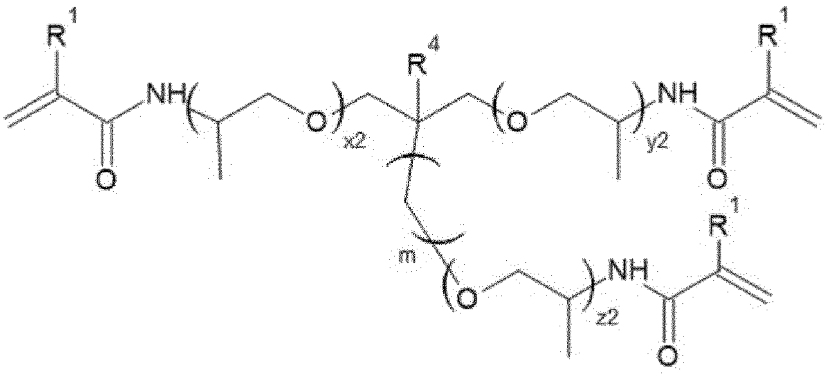

General Formula (3)

[Chemical Formula 4]

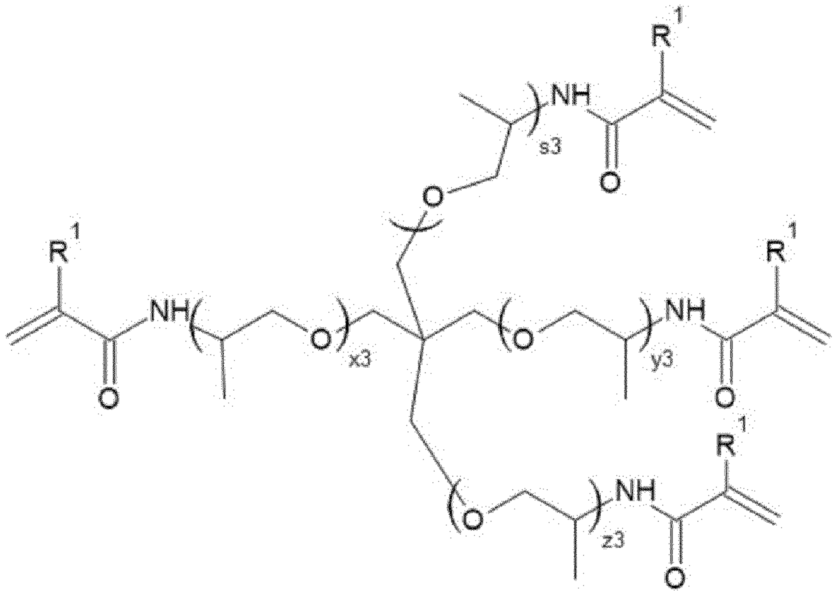

General Formula (4)

(In the formulas (1) to (4), $R^1$ represents a hydrogen atom or a methyl group, may be the same or different. $R^2$ and $R^3$ each represent a divalent chain hydrocarbon group having 3 carbon atoms and may have a linear or branched structure and may be the same or different. $R^4$ represents a hydrogen atom or a chain hydrocarbon group having 1 to 2 carbon atoms. n is an integer from 1 to 70, x1 and z1 are each independently an integer from 1 to 10, y1 is an integer from 1 to 40, x2, y2, and z2 are each independently an integer from 1 to 30, s3, x3, y3, and z3 are each independently an integer from 1 to 20, and m is an integer of 0 or 1.

(7) The curable composition according to any one of (1) to (6), wherein the content of the polyfunctional (meth) acrylamide (A) is 1 to 95% by mass, and the content of the polymerizable compound (B) is 5 to 99% by mass, based on the total mass of the curable composition curable composition.

(8) The curable composition according to any one of (1) to (7), wherein the polymerizable compound (B) has one or more polymerizable groups selected from (meth)acrylate group, (meth)acrylamide group, vinyl group, vinyl ether group, methyl vinyl ether group, allyl group, (meth)allyl ether group, maleimide group, α-substituted maleimide group and α, β-substituted maleimide group.

(9) The curable composition according to any one of the above (1) to (8), wherein the polymerizable compound (B) comprises one or more monofunctional polymerizable compounds (b1) and/or one or more polyfunctional polymerizable compounds (b2), the content of (b1) is from 5 to 80% by mass, and the content of (b2) is 0 to 40% by mass, based on the total mass of the curable composition.

(10) The curable composition according to any one of the above (1) to (9), further comprising a polymerizable polymerization initiator (C) (excluding (A) and (B)), wherein the content of (C) is 0.1 to 20% by mass based on the total mass of the curable composition.

(11) The curable composition according to any one of the above (1) to (10), further comprising a quaternary salt monomer, wherein its content is 0.1 to 30% by mass based on the total mass of the curable composition.

(12) A coating agent composition comprising the curable composition according to any one of (1) to (11).

(13) A pressure-sensitive adhesive composition comprising the curable composition according to any one of (1) to (11).

(14) An adhesive composition comprising the curable composition according to any one of (1) to (11).

(15) An ink composition comprising the curable composition according to any one of (1) to (11).

(16) An aqueous ink composition comprising the curable composition according to any one of (1) to (11).

(17) An ink composition for three-dimensional modeling comprising the curable composition according to any one of (1) to (11).

(18) An aqueous coating composition comprising the curable composition according to any one of (1) to (11).

(19) A sealant composition comprising the curable composition according to any one of (1) to (11).

(20) A nail cosmetic comprising the curable composition according to any one of (1) to (11).

(21) A dental material comprising the curable composition according to any one of (1) to (11).

**(22)** A decorative coating agent comprising the curable composition according to any one of (1) to (11).

[Industrial Applicability]

As described above, the curable composition of the present disclosure comprises one or more multifunctional (meth)acrylamides (A) and one or more polymerizable compounds (B). (A) is amphiphilic and can be mixed with a wide variety of (B) in a wide range of proportions. In addition, if necessary, polymerizable polymerization initiators other than (A) and (B), polymerizable compounds, non-polymerizable components, additives, and the likes can be comprised as needed. The curable compositions of the present disclosure can be suitably used for applications from general-purpose products to various special coating agents, pressure-sensitive adhesives, adhesives, inks, water-based inks, three-dimensional modeling inks, water-based paints, sealants, nail cosmetics, dental materials, decorative coating agents, and the likes.

## Claims

1. A curable composition comprises one or more water-insoluble polyfunctional (meth)acrylamides (A) and one or more polymerizable compounds (B) other than (A).

2. The curable composition according to claim 1, wherein the solubility parameter (SP value) of the polyfunctional (meth) acrylamide (A) is 8.8 to 11.0 $(cal/cm^3)^{1/2}$, the solubility parameter (SP value) of the polymerizable compound (B) is 8.5 to 14.5 $(cal/cm^3)^{1/2}$, and the absolute value of the difference between the SP values of (A) and (B) is 3.0 or less.

3. The curable composition according to claim 1 or 2, wherein the polyfunctional (meth)acrylamide (A) is a compound represented by the general formulas (1) to (4).

[Chemical Formula 1]

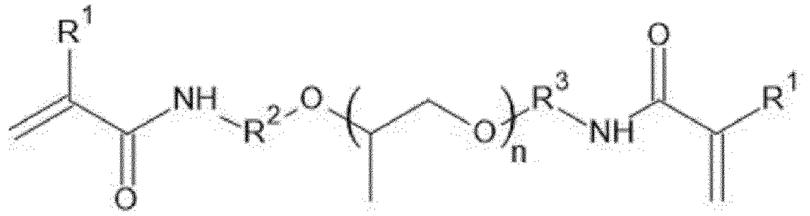

General Formula (1)

[Chemical Formula 2]

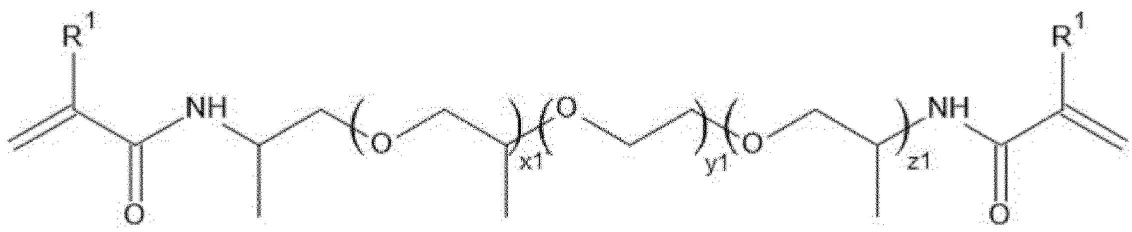

General Formula (2)

[Chemical Formula 3]

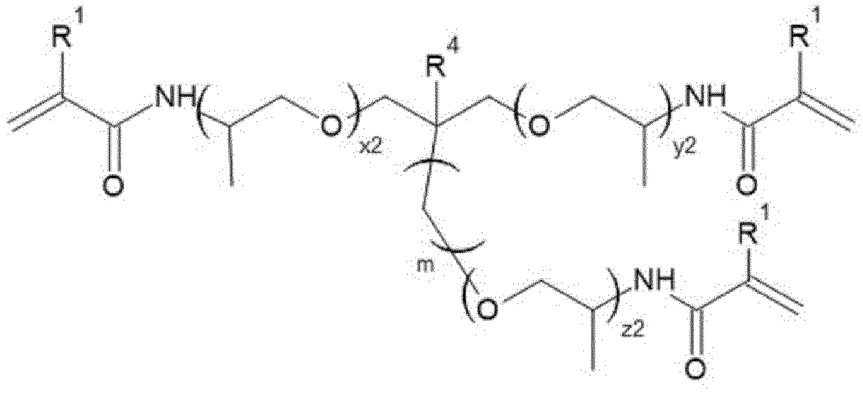

General Formula (3)

[Chemical Formula 4]

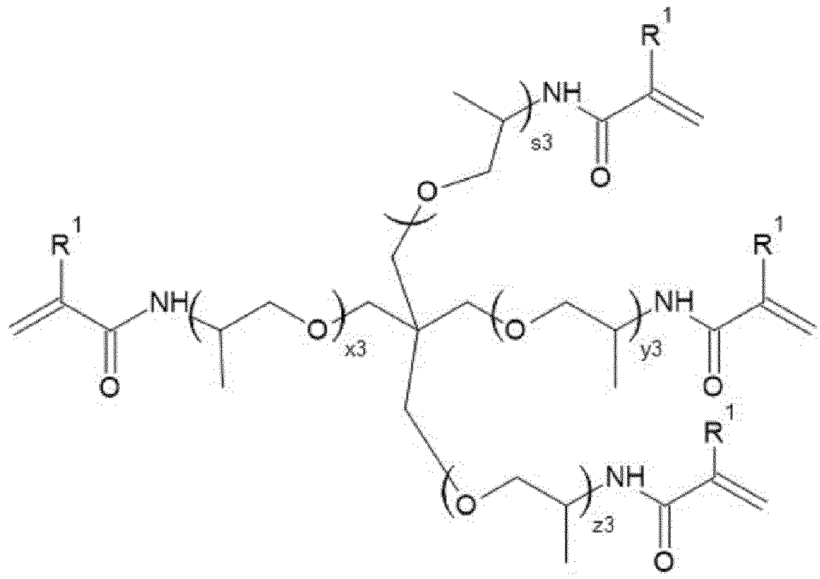

General Formula (4)

(In the formulas (1) to (4), $R^1$ represents a hydrogen atom or a methyl group, may be the same or different. $R^2$ and $R^3$ each represent a divalent chain hydrocarbon group having 3 carbon atoms and may have a linear or branched structure and may be the same or different. $R^4$ represents a hydrogen atom or a chain hydrocarbon group having 1 to 2 carbon atoms. n is an integer from 1 to 70, x1 and z1 are each independently an integer from 1 to 10, y1 is an integer from 1 to 40, x2, y2, and z2 are each independently an integer from 1 to 30, s3, x3, y3, and z3 are each independently an integer from 1 to 20, and m is an integer of 0 or 1.

**4.** The curable composition according to any one of claims 1 to 3, wherein the content of the polyfunctional (meth) acrylamide (A) is 1 to 95% by mass, and the content of the polymerizable compound (B) is 5 to 99% by mass, based on the total weight of the cured composition.

**5.** A coating agent composition comprising the curable composition according to any one of claims 1 to 4.

**6.** A pressure-sensitive adhesive composition comprising the curable composition according to any one of claims 1 to 4.

**7.** An adhesive composition comprising the curable composition according to any one of claims 1 to 4.

**8.** An ink composition comprising the curable composition according to any one of claims 1 to 4.

**9.** An aqueous ink composition comprising the curable composition according to any one of claims 1 to 4.

**10.** An ink composition for three-dimensional modeling comprising the curable composition according to any one of claims 1 to 4.

**11.** An aqueous coating composition comprising the curable composition according to any one of claims 1 to 4.

**12.** A sealant composition comprising the curable composition according to any one of claims 1 to 4.

**13.** A nail cosmetic comprising the curable composition according to any one of claims 1 to 4.

**14.** A dental material comprising the curable composition according to any one of claims 1 to 4.

**15.** A decorative coating agent comprising the curable composition according to any one of claims 1 to 4.

## INTERNATIONAL SEARCH REPORT

International application No.
**PCT/JP2023/012610**

**A. CLASSIFICATION OF SUBJECT MATTER**

***C08F 290/06***(2006.01)i; ***B33Y 70/00***(2020.01)i; ***C08F 220/58***(2006.01)i; ***C09D 4/00***(2006.01)i; ***C09D 4/02***(2006.01)i; ***C09D 11/00***(2014.01)i; ***C09J 4/00***(2006.01)i; ***C09J 4/02***(2006.01)i; ***C09J 11/06***(2006.01)i
FI: C08F290/06; C08F220/58; C09J4/02; C09J4/00; C09J11/06; C09D4/02; C09D4/00; C09D11/00; B33Y70/00

According to International Patent Classification (IPC) or to both national classification and IPC

**B. FIELDS SEARCHED**

Minimum documentation searched (classification system followed by classification symbols)

C08F290/06; B33Y70/00; C08F220/58; C09D4/00; C09D4/02; C09D11/00; C09J4/00; C09J4/02; C09J11/06

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 1-163166 A (SOKEN CHEMICAL & ENGINEERING CO., LTD.) 27 June 1989 (1989-06-27)<br>claims, p. 3, upper right column, line 6 from the bottom to the last line | 1-6, 8-9, 11 |
| A | | 7, 10, 12-15 |
| X | JP 2013-43946 A (FUJIFILM CORP.) 04 March 2013 (2013-03-04)<br>claims, paragraphs [0030]-[0034], examples, table 1 | 1-2, 4-6, 8-10 |
| A | | 3, 7, 11-15 |
| X | WO 2013/008626 A1 (FUJIFILM CORP.) 17 January 2013 (2013-01-17)<br>claims, examples, in particular, paragraphs [0123]-[0130], table 1 | 1-2, 4-6, 8-10 |
| A | | 3, 7, 11-15 |
| X | JP 2020-100602 A (KURARAY NORITAKE DENTAL INC.) 02 July 2020 (2020-07-02)<br>claims, examples | 1-2, 4-7, 14 |
| A | | 3, 8-13, 15 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 May 2023** | **06 June 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2023/012610**

| Patent document cited in search report | Publication date (day/month/year) | Patent family member(s) | Publication date (day/month/year) |
|---|---|---|---|
| JP 1-163166 A | 27 June 1989 | (Family: none) | |
| JP 2013-43946 A | 04 March 2013 | US 2013/0050365 A1<br>claims, paragraphs [0050]-[0052], examples, table 1<br>CN 102952429 A | |
| WO 2013/008626 A1 | 17 January 2013 | US 2014/0132685 A1<br>claims, examples, in particular, paragraphs [0217]-[0231], table 1<br>EP 2730625 A1<br>CN 103635545 A | |
| JP 2020-100602 A | 02 July 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2010208964 A


### Non-patent literature cited in the description


- *Polymer Engineer Science*, 1974, vol. 14, 147